(19) Europäisches Patentamt
European Patent Office
Office européen des brevets

(11) Publication number: **0 187 355**
A1

(12) # EUROPEAN PATENT APPLICATION

(21) Application number: 85116364.2

(22) Date of filing: 23.09.81

(51) Int. Cl.[4]: C 07 D 205/08
C 07 D 417/12, C 07 C 143/86
C 07 C 125/065, C 07 D 405/14
C 07 D 403/12, C 07 D 409/12

(43) Date of publication of application:
16.07.86 Bulletin 86/29

(84) Designated Contracting States:
IT

(60) Publication number of the earlier application in accordance with Art. 76 EPC: 0 048 953

(71) Applicant: E.R. Squibb & Sons, Inc.
Lawrenceville-Princeton Road
Princeton, N.J. 08540(US)

(72) Inventor: Fritz, Alan William
9 Beck Court
Kendall Park New Jersey(US)

(72) Inventor: Floyd, David Mack
R.R. 1, Box 404 M
Pennington New Jersey(US)

(72) Inventor: Sykes, Richard Brook
530 River Road
Belle Mead New Jersey(US)

(72) Inventor: Parker, William Lawrence
216 Hale St.
Pennington New Jersey(US)

(72) Inventor: Cimarusti, Christopher Michael
5 Kingsberry Drive
Somerset New Jersey(US)

(72) Inventor: Koster, William Henry
10 Mallard Drive
Pennington New Jersey(US)

(72) Inventor: Slusarchyk, William Allen
136 Sunset Rd, R.D. 3
Belle Mead New Jersey(US)

(74) Representative: Jacobacci, Filippo et al,
c/o JACOBACCI-CASETTA & PERANI S.n.c. Via Alfieri, 17
I-10121 Torino(IT)

(54) **Process for preparing beta-lactam antibiotics.**

(57) β-lactams having a sulfonic acid substituent in the 1-position and one or two substituents as specified in the 4-position and an amine function in the 3-position, are prepared by sulfonating a corresponding β-lactam having a hydrogen substituent in the 1-position and a protected amino substituent in the 1-position and removing said amino protecting group.

EP 0 187 355 A1

Croydon Printing Company Ltd.

# PROCESS FOR PREPARING β-LACTAM ANTIBIOTICS

This invention is directed to processes for preparing a novel family of ß-lactam antibiotics, useful as antibacterial agents.

β-Lactams having a sulfonic acid salt (including "inner salt") substituent in the 1-position and an acylamino substituent in the 3-position and having one or two alkyl, cycloalkyl, phenyl or substituted phenyl substituents in the 4-position or a single alkoxycarbonyl, alken-1-yl, alkyn-1-yl, 2-phenylethenyl or 2-phenylethynyl substituent in the 4-position exhibit activity against a range of gram-negative and gram-positive bacteria.

The preferred members of the novel family of ß-lactam antibiotics of this invention are those encompassed by the formula

I

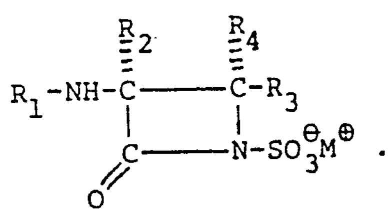

In addition to the above described β-lactams an acylamino substituent in the 3-position, this invention also encompasses counterpart compounds having an amino substituent in the 3-position.

The preferred compounds of this type have the formula

Ia

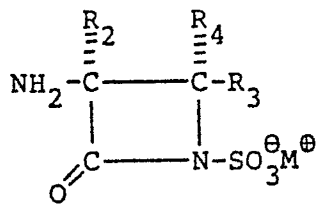

These compounds are intermediates useful for the preparation of corresponding 3-acylamino compounds.

As used in formulas I and Ia, and throughout the specification, the symbols are as defined below:

$R_1$ is acyl;

$R_2$ is hydrogen or alkoxy of 1 to 4 carbons;

$R_3$ and $R_4$ are the same or different and each is alkyl, cycloalkyl, phenyl or substituted phenyl, or one of $R_3$ and $R_4$ is hydrogen and the other is alkyl, cycloalkyl, phenyl, substituted phenyl, alkoxycarbonyl, alken-1-yl, alkyn-1-yl, 2-phenylethenyl or 2-phenylethynyl.

$M^{\oplus}$ is hydrogen or a cation.

The terms "alkyl" and "alkoxy" refer to both straight and branched chain groups. Those groups having 1 to 10 carbon atoms are preferred.

The terms "cycloalkyl" and "cycloalkenyl" refer to cycloalkyl and cycloalkenyl groups having 3,4,5,6 or 7 carbon atoms.

The term "alkenyl" refers to both straight and branched chain groups. Those groups having 2 to 10 carbon atoms are preferred.

The term "halogen" refers to fluorine, chlorine, bromine and iodine.

The term "substituted phenyl" refers to a phenyl group substituted with 1,2 or 3 amino, halogen, hydroxyl, trifluoromethyl and lower alkyl or alkoxy groups.

The term "protected carboxyl" refers to a carboxyl group which has been esterified with a conventional ester protecting group. These groups are well known in the art; see, for example, United States patent 4,144,333, issued March 13, 1979. The preferred protected carboxyl groups are benzyl, benzhydryl and t-butyl esters.

The term "acyl" includes all organic radicals derived from an organic acid (i.e., a carboxylic acid) by removal of the hydroxyl group. Certain acyl groups are, of course, preferred, but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see, for example, Cephalosporins and Penicillins, edited by Flynn, Academic Press (1972), German Offenlegungsschrift 2,716,677 published October 10, 1978, Belgian patent 867,994, published December 11, 1978, United States patent 4,152,432, issued

May 1, 1979, United States patent 3,971,778, issued July 27, 1976, United States patent 4,172,199, issued October 23, 1979, and British patent 1,348,894, published March 27, 1974.

The following list of acyl groups is presented to further exemplify the term "acyl"; it should not be regarded as limiting that term. Exemplary acyl groups are:

(a) Aliphatic groups having the formula

$$R_5-\overset{O}{\overset{\parallel}{C}}-$$

wherein $R_5$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

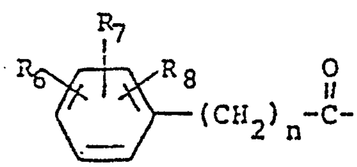 ,

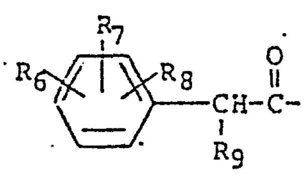

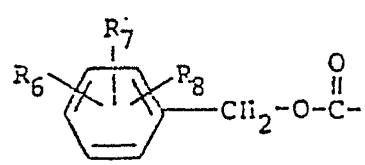 ,

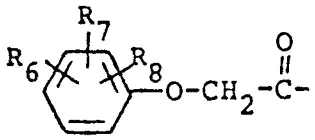,

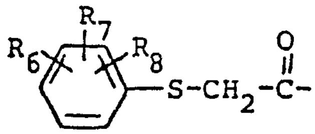 or

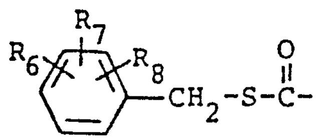

wherein n is 0, 1, 2 or 3; $R_6$, $R_7$, and $R_8$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_9$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

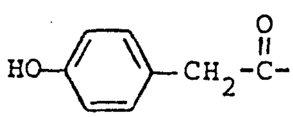 ,

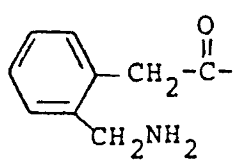 ,

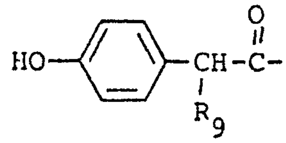 ($R_9$ is preferably a carboxyl salt or sulfo salt) and

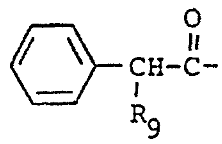 ($R_9$ is preferably a carboxyl salt or sulfo salt).

(c) Heteroaromatic groups having the formula

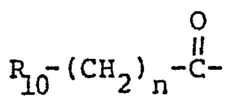,

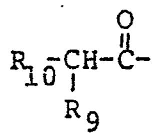,

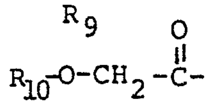,

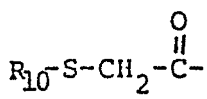, or

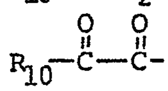,

wherein n is 0, 1, 2 or 3; $R_9$ is as defined above; and $R_{10}$ is a substituted or unsubstituted 5-,6- or 7-membered heterocyclic ring containing 1, 2, 3 or 4 (preferably 1 or 2) nitrogen, oxygen and sulfur atoms. Exemplary heterocyclic

rings are thienyl, furyl, pyrrolyl, pyridinyl, pyrazinyl, thiazolyl, morpholinyl, pyrimidinyl and tetrazolyl. Exemplary substituents are halogen, hydroxyl, nitro, amino, cyano, trifluoromethyl, alkyl of 1 to 4 carbon atoms, or alkoxy of 1 to 4 carbon atoms.

Preferred heteroaromatic acyl groups include those groups of the above formulas wherein $R_{10}$ is 2-amino-4-thiazolyl, 2-amino-5-halo-4-thiazolyl, 4-aminopyrimidin-2-yl, 5-amino-1,2,4-thiadiazol-5-yl, 2-thienyl or 2-furanyl.

(d) [[(4-Substituted-2,3-dioxo-1-piperazinyl)-carbonyl]amino]arylacetyl groups having the formula

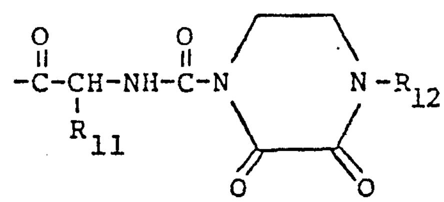

wherein $R_{11}$ is an aromatic group (including carbocyclic aromatics such as those of the formula

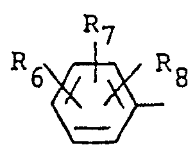

and heteroaromatics as included within the definition of $R_{10}$); and $R_{12}$ is alkyl, substituted alkyl (wherein the alkyl group is substituted with one or more halogen, cyano, nitro, amino or mercapto groups), arylmethyleneamino (*i.e.*, $-N=CH-R_{11}$ wherein $R_{11}$ is as defined above), arylcarbonylamino (*i.e.*, $-NH-\overset{O}{\overset{\|}{C}}-R_{11}$ wherein $R_{11}$ is as defined above) or alkylcarbonylamino.

Preferred [[(4-substituted-2,3-dioxo-1-piperazinyl)carbonyl]amino]arylacetyl groups include those wherein $R_{12}$ is ethyl, phenylmethyleneamino or 2-furylmethyleneamino.

(e) (Substituted oxyimino)arylacetyl groups having the formula

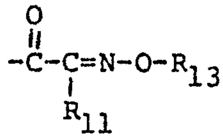

wherein $R_{11}$ is as defined above and $R_{13}$ is hydrogen, alkyl, cycloalkyl, alkylaminocarbonyl, arylaminocarbonyl (i.e., 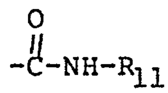 wherein $R_{11}$ is as defined above) or substituted alkyl (wherein the alkyl group is substituted with 1 or more halogen, cyano, nitro, amino, mercapto, alkylthio, aromatic group (as defined by $R_{11}$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy (phenylmethoxy)phosphinyl, or dialkoxyphosphinyl substituents).

Preferred (substituted oxyimino)arylacetyl groups include those wherein $R_{11}$ is 2-amino-4-thiazolyl. Also preferred are those groups wherein $R_{13}$ is methyl, ethyl, carboxymethyl, or 2-carboxyisopropyl.

(f) (Acylamino)arylacetyl groups having the formula

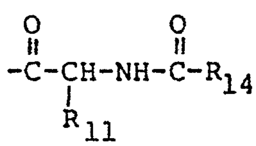

wherein $R_{11}$ is as defined above and $R_{14}$ is

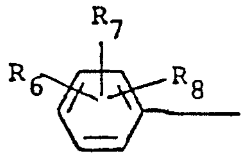 $(CH_2)_n$-O-, amino, alkylamino, (cyanoalkyl)amino, amido, alkylamido, (cyanoalkyl)-amido, 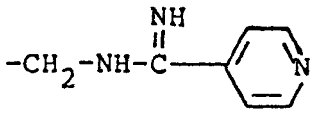, 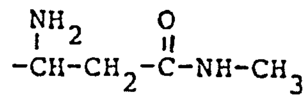,

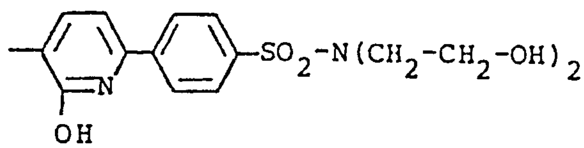, 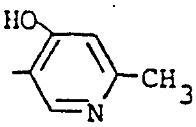,

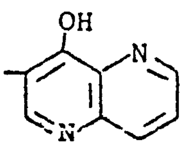, or 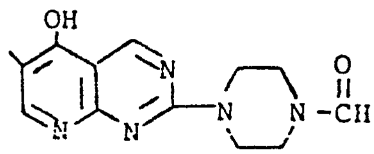.

Preferred (acylamino)arylacetyl groups of the above formula include those groups wherein $R_{14}$ is amino, or amido. Also preferred are those groups wherein $R_{11}$ is phenyl or 2-thienyl.

(g) [[[3-Substituted-2-oxo-1-imidazolidinyl]carbonyl]amino]arylacetyl groups having the formula

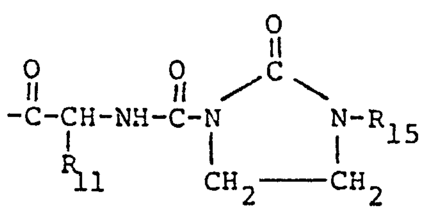

wherein $R_{11}$ is as defined above and $R_{15}$ is hydrogen, alkylsulfonyl, arylmethyleneamino (i.e., $-N{=}CH{-}R_{11}$ wherein $R_{11}$ is as defined above), $-\overset{O}{\overset{\|}{C}}-R_{16}$ (wherein $R_{16}$ is hydrogen, alkyl or halogen substituted alkyl), aromatic group (as defined by $R_{11}$ above), alkyl or substituted alkyl (wherein the alkyl group is substituted with one or more halogen,cyano, nitro, amino or mercapto groups).

Preferred [[3-substituted-2-oxo-1-imidazolidinyl]carbonyl]amino]arylacetyl groups of the above formula include those wherein $R_{11}$ is phenyl or 2-thienyl. Also preferred are those groups wherein $R_{15}$ is hydrogen, methylsulfonyl, phenylmethyleneamino or 2-furylmethyleneamino.

The term "cation", as used throughout the specification, refers to any positively charged atom or group of atoms. The "$-SO_3^{\ominus}M^{\oplus}$" substituent on the nitrogen atom of the β-lactams of this invention encompasses all sulfonic acid salts. Pharmaceutically acceptable salts are, of course, preferred, although other salts are also useful in purifying the products of this invention or as intermediates for the preparation of pharmaceutically acceptable salts. The cationic portion of the sulfonic acid salts of this invention can be obtained from either organic or inorganic bases. Such cationic portion includes, but is not limited to, the following ions: ammonium; substituted ammonium, such as alkylammonium (e.g., tetra-n-butylammonium, referred to hereinafter as tetrabutylammonium); alkali metal, such as lithium, sodium and potassium; alkaline earth metal, such as calcium and magnesium;

pyridinium; dicyclohexylammonium; hydrabaminium; benzathinium; N-methyl-D-glucaminium.

Some of the compounds of this invention may be crystallized or recrystallized from solvents containing water. In these cases water of hydration may be formed. This invention contemplates stoichiometric hydrates as well as compounds containing variable amounts of water that may be produced by processes such as lyophilization.

β-Lactams having a sulfonic acid salt substituent in the 1-position and an amino or acylamino substituent in the 3-position contain at least one chiral center — the carbon atom (in the 3-position of the β-lactam nucleus) to which the amino or acylamino substituent is attached. This invention is directed to those β-lactams which have been described above, wherein the stereochemistry at the chiral center in the 3-position of the β-lactam nucleus is the same as the configuration at the carbon atom in the 6-position of naturally occurring penicillins (e.g., penicillin G) and as the configuration at the carbon atom in the 7-position of naturally occurring cephamycins (e.g., cephamycin C).

With respect to the preferred β-lactams of formulas I and Ia, the structural formulas have been drawn to show the stereochemistry at the chiral center in the 3-position. Because of the nomenclature convention, those compounds of formulas I and Ia wherein $R_2$ is hydrogen have the S configuration and those compounds of formulas I and Ia wherein $R_2$ is alkoxy have the R configuration.

Also included within the scope of this invention are racemic mixtures which contain the above-described β-lactams.

The compounds of this invention have activity against a range of gram-negative and gram-positive organisms and can be used as agents to combat bacterial infections (including urinary tract infections and respiratory infections) in mammalian species, such as domesticated animals (e.g., dogs, cats, cows, horses, and the like) and humans.

For combating bacterial infections in mammals a compound of this invention can be administered to a mammal in need thereof in an amount of about 1.4 mg/kg/day to about 350 mg/kg/day, perferably about 14 mg/kg/day to about 100 mg/kg/day. All modes of administration which have been used in the past to deliver penicillins and cephalosporins to the site of the infection are also contemplated for use with the novel family of β-lactams of this invention. Such methods of administration include oral, intravenous, intramuscular and as a suppository.

The β-lactam products of this invention are generally prepared by the introduction of a sulfonic acid substituent (a sulfo group $-SO_3-$) onto the nitrogen atom in the 1-position of the β-lactam nucleus. This sulfonation reaction is readily effected by treating the β-lactam with a sulfur trioxide complex or with an equivalent sulfonating reagent such as a chlorosulfonate.

The sulfur trioxide complexes most commonly used are pyridine-sulfur trioxide; lutidine-sulfur trioxide; dimethylformamide-sulfur trioxide; and picoline-sulfur trioxide. Instead of using a pre-formed complex, the complex can be formed in situ, e.g., using chlorosulfonyl-trimethylsilyl ester and pyridine as reagents. Alternatively, sulfonation can be effected by way of an intermediate compound as for example first silylating the nitrogen atom of the β-lactam nucleus and then subjecting the silated compound to a silyl interchange reaction with trimethylsilylchlorosulfonate or a similar reagent. Exemplary silylating agents are monosilyltrifluoroacetamide, trimethylsilylchloride/triethylamine and bis-trimethylsilyl trifluoroacetamide.

Generally, the sulfonation reaction is carried out in the presence of an organic solvent such as pyridine or a mixture of organic solvents, preferably a mixture of a polar solvent such as dimethylformamide and a halogenated hydrocarbon such as dichloromethane.

The product initially formed in the sulfonation reaction is a salt of the sulfonated β-lactam. When pyridine-sulfur trioxide is the sulfonating complex the product initially formed is the β-lactam-sulfonated pyridinium salt of the sulfonated β-lactam wherein $M^+$ in the formula below is the pyridinium ion:

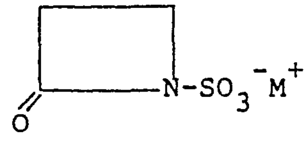

These complexes can be converted to other sulfonic acid salts using conventional techniques (e.g., ion-exchange resins, crystallization or ion-pair extraction. These conversion techniques are also useful in purifying the products. Conversion of the pyridine salt to the potassium salt using potassium phosphate or potassium ethyl hexanoate; to the tetrabutylammonium salt using tetrabutyl ammonium hydrogen sulfate; or to a zwitterian ($M^+$ = hydrogen) using formic acid; are particularly useful.

It should be appreciated that the sulfonation reaction which introduces the sulfo group onto the nitrogen atom of the β-lactam nucleus can be effected at various stages of the synthesis, including insertion prior to the formation of a β-lactam nucleus, where such procedure is followed as outlined below. The sulfonation reaction is effected in the presence of solvents previously described and usually at room temperature. Where the amino function is present it is preferably conducted with the amino function protected.

Using a benzyloxycarbonyl protecting group as an example, the sulfonation reaction can be visualized as follows:

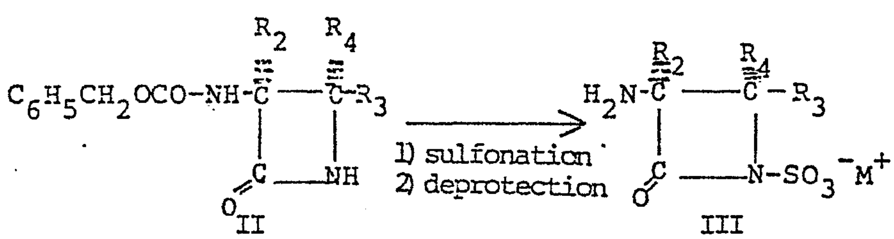


II III

Other protecting groups can be used to protect the amine function, for example, a t-butyloxycarbonyl group, a simple acyl group such as acetyl or benzoyl of phenylacetyl, a triphenylmethyl group, or having the amino function in the form of an azide group. The desired acyl group ($R_1$) can then be affixed by a conventional acylation reaction.

Exemplary acylation techniques for converting a compound of formula III to a product of formula I include reaction with a carboxylic acid ($R_1$-OH), or corresponding carboxylic acid halide or carboxylic acid anhydride. When $R_2$ is alkoxy, acylcation is best effected by use of an acid chloride or acid bromide. The reaction with a carboxylic acid proceeds most readily in the presence of a carbodiimide such as dicyclohexylcarbodiimide and a substance capable of forming an active ester in situ such as N-hydroxybenzotriazole. In those instances when the acyl group ($R_1$) contains reactive functionality (such as amino or carboxyl groups) it may be necessary to first protect those functional groups, then carry out the acylation reaction, and finally deprotect the resulting product. Alternatively, the sulfonation reaction can be effected with the acyl group already in place, i.e.,

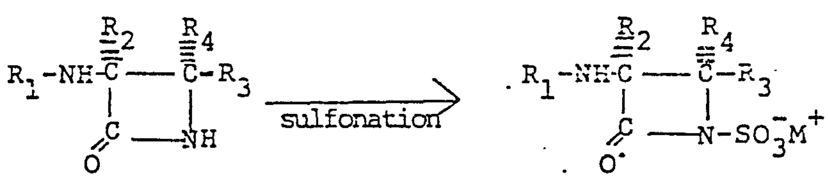


IV V

In the case where $R_2$ is lower alkoxy, there can be an additional variation in that the $R_2$-lower alkoxy group can be inserted after the sulfonation as well as before the sulfonation using the conventional procedure of chlorinating the acylated nitrogen atom in the 3-position followed by reaction with a lower alkoxide

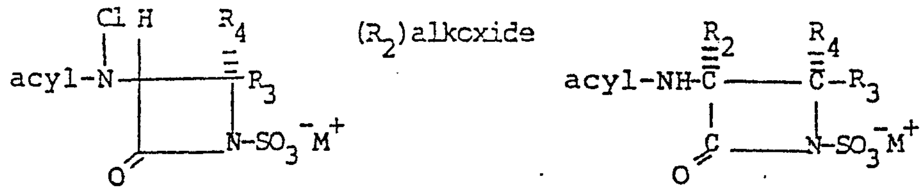


VI V

The acyl groups in the above reaction also include easily removable groups which function as a protecting group and which can be removed after the reaction to afford the "deacylated" product ($-NH_2$).

In addition, the β-lactam ring can be formed via a cyclization reaction and the sulfonation reaction can be effected prior to cyclization as well as subsequent thereto, i.e.,

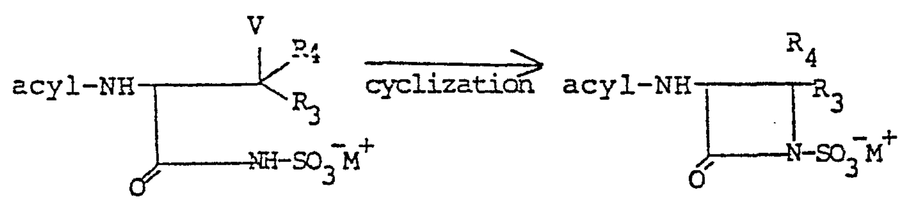


VII

The acyl group in this reaction can also be an easily removable group which functions as a protecting group and which on removal affords the $-NH_2$ product.

V in the above formula VII is a leaving group.

The azetidinone starting materials wherein $R_2$ is hydrogen and one of $R_3$ and $R_4$ is hydrogen can also be prepared from amino acids having the formula

XII 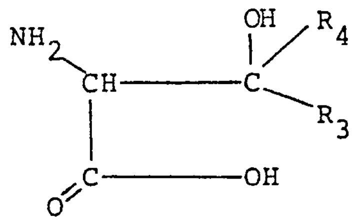

(one of $R_3$ and $R_4$ is hydrogen). The amino group is first protected with a classical protecting group, e.g., t-butoxycarbonyl (referred to hereinafter as "Boc"). The carboxyl group of the protected amino acid is then reacted with an amine salt having the formula

XIII $Y-O-\overset{+}{N}H_3\overset{-}{Cl}$

wherein Y is alkyl or benzyl, in the presence of a carbodiimide to yield a compound having the formula

XIV 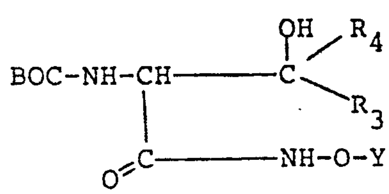

( one of $R_3$ and $R_4$ is hydrogen). The hydroxyl group of a compound of formula XIV is converted to a leaving group (V) with a classical reagent, e.g., methanesulfonyl chloride (methanesulfonyl is referred to hereinafter as "Ms"). Other leaving groups (V) which can be utilized are benzenesulfonyl, toluenesulfonyl, chloro, bromo and iodo.

The fully protected compound having the formula

XV 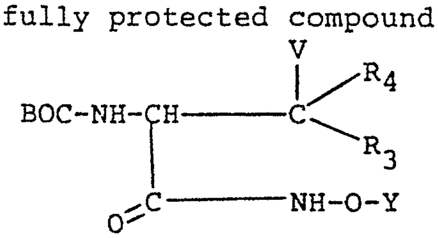

(one of $R_3$ and $R_4$ is hydrogen) is cyclized by treatment with base, e.g., potassium carbonate. The reaction is preferably carried out in an organic solvent such as acetone, under reflux conditions, and yields a compound having the formula

XVI 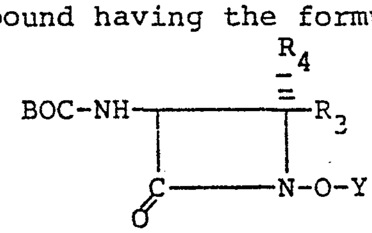

(one of $R_3$ and $R_4$ is hydrogen).

Alternatively, cyclization of a compound of formula XIV can be accomplished without first converting the hydroxyl group to a leaving group. Treatment of a compound of formula XIV with triphenylphosphine and diethylazodicarboxylate, yields a compound of formula XVI wherein one of $R_3$ and $R_4$ is hydrogen.

Removal of the protecting group from the 1-position of an azetidinone of formula XVI can be accomplished via sodium reduction when Y is alkyl, and yields an intermediate having the formula

XVII

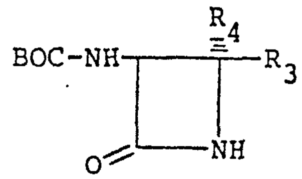

(one of $R_3$ and $R_4$ is hydrogen). If Y is benzyl, catalytic (e.g., palladium on charcoal) hydrogenation will initially yield the corresponding N-hydroxy compound, which upon treatment with titanium trichloride yields an intermediate of formula XVII wherein one of $R_3$ and $R_4$ is hydrogen.

Synthesis involving ring closure of the type described above results in inversion of the stereochemical configuration of the R3 and R4 substituents.

As previously mentioned, the above azetidinone can be sulfonated to form a compound having the formula

XVIII

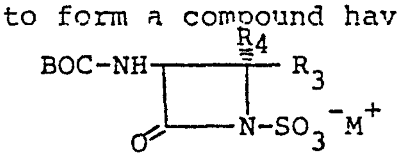

(one of $R_3$ and $R_4$ is hydrogen).

An alternative procedure for preparing a compound of formula I wherein $R_2$ is hydrogen and one of $R_3$ and $R_4$ is hydrogen, utilizes as a starting material an amino acid amide having the formula

XIX

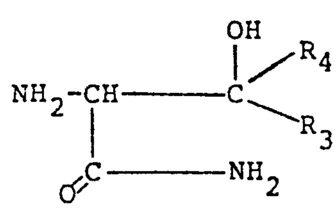

(one of $R_3$ and $R_4$ is hydrogen).

Protection of the amino group with a classical protecting group, e.g., benzyloxycarbonyl (reffered to hereinafter as Z) or Boc, and conversion of the hydroxyl group to a leaving group (V) such as Ms yields a compound having the formula

XX 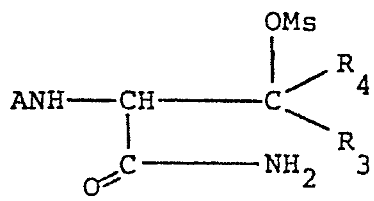

(one of $R_3$ and $R_4$ is hydrogen), wherein A is a protecting group.

Sulfonation of a compound of formula XX yields a compound having the formula

XXI 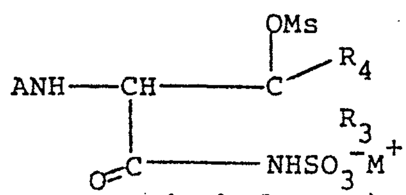

(one of $R_3$ and $R_4$ is hydrogen).

Cyclization of a compound of formula XXI is accomplished with base, e.g., potassium carbonate. The reaction is preferably carried out in a mixture of water and an organic solvent (e.g., a halogenated hydrocarbon such as 1,2-dichloroethane) under reflux conditions and yields a compound having the formula

XXII 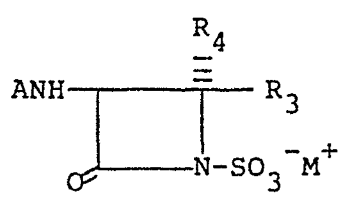

(one of $R_3$ and $R_4$ is hydrogen).

Deprotection of a sulfonated azetidinone of formula XXII , wherein A is a protecting group, as well as the counterpart compounds previously described having an $R_2$-alkoxy group by catalytic hydrogenation yields a compound having the formula

XXIII 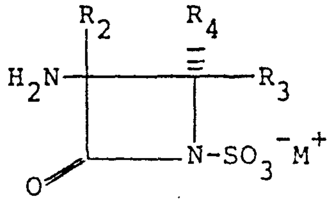

(one of $R_3$ and $R_4$ is hydrogen), wherein $R_2$ is hydrogen or alkoxy which can be converted to the corresponding zwitterion having the formula

XXIV 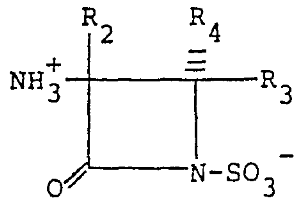

(one of $R_3$ and $R_4$ is hydrogen) by treatment with an acid such as formic acid.

Deprotection of a sulfonated azetidinone of formula XXII wherein A is a Boc protecting group using acidic conditions (e.g., using formic acid) yields the corresponding zwitterion of formula XIX.

A 3-azido-2-azetidinone starting material can be prepared by first reacting an olefin having the formula

XXV 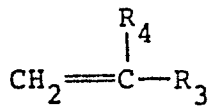

with a halosulfonylisocyanate (preferably chlorosulfonylisocyanate) having the formula

XXVI $O{=}C{=}N{-}SO_2$-halogen

to yield an azetidinone having the formula

XXVII 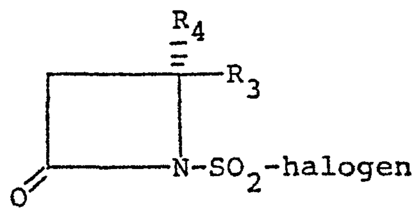

Reductive hydrolysis of an azetidinone of formula XXVII yields an N-unsubstituted β-lactam having the formula

XXVIII 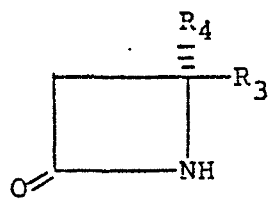

For a more detailed description of the above described reaction sequence reference can be made to the literature; see, for example, Chem. Soc. Rev., 5, 181 (1976) and J. Org. Chem., 35, 2043 (1970).

An azido group can be introduced in the 3-position of an azetidinone of formula XXVIII (or the sulfonated counterpart) by reaction of the compound with an arylsulfonyl azide (such as toluenesulfonyl azide) to obtain a starting azetidinone having the formula

XXIX 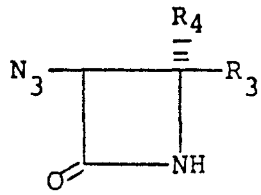

The reaction proceeds best by first protecting the azetidinone nitrogen with a silyl residue (e.g., t-butyldimethylsilyl, or t-butyldiphenylsilyl), then generating the anion at the 3-position of the nucleus with a strong organic base (e.g., lithium diisopropylamine) at a low temperature, and then treating the anion with toluenesulfonyl azide. The resulting intermediate is quenched with trimethylsilyl chloride, and subsequent acid hydrolysis or fluoride solvolysis of the N-protecting group yields the compound of formula XXIX.

Alternatively, the compound of Formula XXXI can be obtained by first reacting a primary amine having the formula

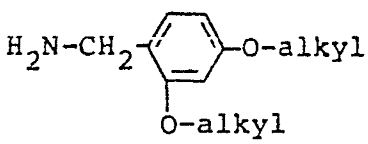 or 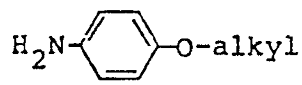

with an aldehyde of the formula $R_3CH=O$ to yield the corresponding Schiff base. A[2+2]cycloaddition with an activated form of azido acetic acid yields a 3-azido-2-azetidinone having the formula

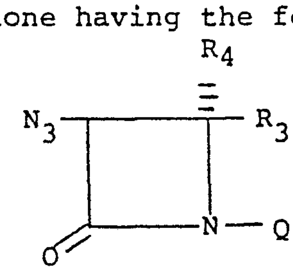

wherein Q is

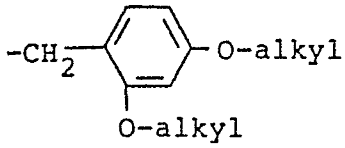

or

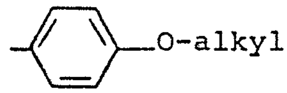

Oxidative removal of the Q-substituent yields the compound of formula XXIX.

The 3-acylamino-2-azetidinones can be obtained by first reducing a 3-azido-2-azetidinone of formula XXIX. to obtain the corresponding 3-amino-2-azetidinone and then acylating the 3-amino-2-azetidinone.

As previously mentioned in the case where $R_2$ is lower alkoxy, the product can be prepared from the counterpart product wherein $R_2$ is hydrogen. Clorination of the amide nitrogen of a nonalkoxylated compound yields an intermediate having the formula

XXX

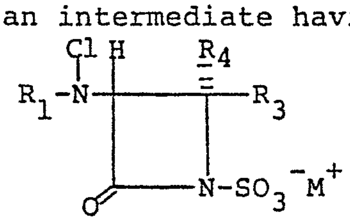

Reagents and procedures for N-chlorinating amides are known in the art. Exemplary reagents are tert.-butyl hypochlorite, sodium hypochlorite, and chlorine. The reaction can be run in an organic solvent (e.g., a lower alkanol such as methanol) or in a two phase solvent system (e.g., water/methylene chloride) in the presence of a base such as sodium borate decahydrate. The reaction is preferably run at a reduced temperature.

Reaction of an intermediate of formula XXIX with an alkoxylating agent, e.g., an alkali metal alkoxide yields a product of formula I wherein $R_2$ is alkoxy, in combination with its enantiomer. The reaction can be run in an organic solvent, e.g., a polar organic solvent such as dimethylformamide, at a reduced temperature.

An alternative synthesis for preparing the compounds of formula I wherein $R_2$ is alkoxy comprises first alkoxylating an intermediate of formula VI wherein $R_1NH$ is a carbamate (e.g., $R_1$ is benzyloxycarbonyl) and $R_2$ is hydrogen and then introducing a sulfo group in the 1-position of the resulting compound. Chlorination of a compound of formula VI using the procedure described above (for chlorination of a non-alkoxylated compound of formula I to yield a compound of formula XXX yields an intermediate having the formula

XXXI

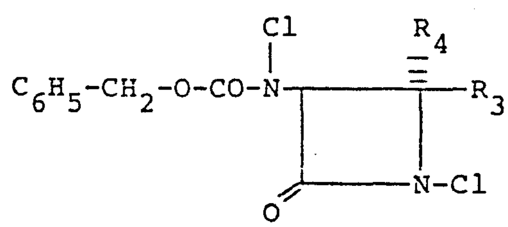

Using the alkoxylation procedure described above (for converting a compound of formula XXX to a product of formula I), and subsequently adding a reducing agent such as trimethylphosphite, the compound of formula XXXI can be converted to an intermediate having the formula

XXXII

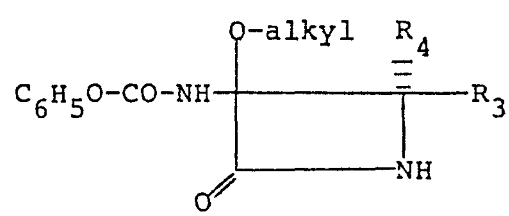

in a combination with its enantiomer.

The above procedures yield those products of formula I wherein $R_2$ is alkoxy as a racemic mixture. If desired the enantiomer having the R configuration can be isolated from the racemic mixture using conventional techniques such as fractional crystallization of a suitable salt with an optically active organic amine or by ion-paired chromatography utilizing an optically active cation.

## Example 1

## (±—cis)-4-Methyl-2-oxo-3-[[(phenylmethoxy)carbonyl]-amino]-1-azetidinesulfonic acid, potassium salt

A) N-Benzyloxy-t-boc*-allothreonine amide

A solution of 6.9 g of d,l-t-boc-allothreonine and the free amine from 5.3 g of o-benzylhydroxylamine·HCl (∿0.033 mole, ethyl acetate-sodium bicarbonate liberation) in 80 ml of tetrahydrofuran is treated with 4.82 g of N-hydroxybenzotriazole and 6.5 g of dicyclohexylcarbodiimide in 20 ml of tetrahydrofuran. After stirring for about 16 hours at room temperature the slurry is filtered, concentrated *in vacuo* and chromatographed on a 400 ml of silica gel column. Elution with 5-10% ethyl acetate in chloroform gives 6.8 g of the title compound in fractions (200 ml each) 7-22.

B) (±—cis)-N-Benzyloxy-3-t-butoxycarbonylamino-4-methylazetidinone

A solution of 6.8 g of N-benzyloxy-t-boc-allothreonine amide in 200 ml of tetrahydrofuran is stirred for about 16 hours with 5.24 g of triphenylphosphine and 3.2 ml of diethylazodicarboxylate. The solvents are evaporated *in vacuo* and the residue is chromatographed on a 500 ml silica gel column. Elution with methylene chloride followed by crystallization from ether gives a total of 2.65 g of azetidinone. Rechromatography of the mother liquors and mixed fractions give an

* "boc" is used to describe butoxycarbonyl

additional 0.6 g. Crystallization of a portion twice from ether (-20°C) gives the analytical sample of the title compound melting point 140-142°C.

C) (±-cis)-3-t-Butoxycarbonylamino-1-hydroxy-4-methylazetidinone

A solution of 3.2 g of cis-N-benzyloxy 3-t-butoxycarbonylamino-4-methylazetidinone in 200 ml of 95% ethanol is stirred in an atmosphere of hydrogen with 0.7 g of 10% palladium on charcoal. After 40 minutes the slurry is filtered (uptake 249 ml) and the filtrate is evaporated and triturated with ether to give, in two crops, 2.05 g of solid, melting point 134-136°C.

D) (±-cis-3-t-Butoxycarbonylamino-4-methylazetidinone

A solution of 2.05 g of cis-3-t-butyloxycarbonylamino-1-hydroxy-4-methylazetidinone in 60 ml of methanol is treated with a total of 90 ml of 4.5 M ammonium acetate (40, 20 and 30 ml portions) and 45 ml of 1.5 M titanium trichloride (20, 10 and 15 ml portions) the second and third additions are made after 15 and 120 minutes, respectively. After 135 minutes the solution is diluted with an equal volume of 8% sodium chloride and extracted with three 300 ml portions of ethyl acetate. The combined organic layer is washed with a mixture of 100 ml each of 5% sodium bicarbonate and saturated salt, dried, and evaporated. Trituration with ether gives, in two crops, 1.65 g of solid. A portion of the first crop is recrystallized from ether to give the analytical sample, melting point 176-178.5°C.

E) (±-*cis*)-3-Benzyloxycarbonylamino-4-methylazetidinone

A solution of 1.55 g of *cis*-3-*t*-butoxycarbonylamino-4-methylazetidinone in 4 ml each of methylene chloride and anisole is cooled to 0°C and 50 ml of cold trifluoroacetic acid is added. After 90 minutes the solvents are evaporated *in vacuo* (benzene added and evaporated three times). The residue is dissolved in 25 ml of acetone, the initial pH (2.5) is raised to 7 with 5% sodium bicarbonate, and 2 ml of benzylchloroformate is added. The solution is kept at 0°C and pH 7 for 4 hours and the acetone is removed *in vacuo* to give a slurry that is filtered. The filtrate is saturated with salt and extracted with methylene chloride. The solid is dissolved in methylene chloride and dried. The organic layers are combined, concentrated, and the residue chromatographed on a 200 ml silica gel column. Elution with 3:1 chloroform, ethyl acetate gives 850 mg of the title compound in fractions (100 ml each) 4-11. Crystallization of a small sample from ether gives the analytical sample, melting point 165-166°C.

F) (±-*cis*)-4-Methyl-2-oxo-3-[[(phenylmethoxy)-carbonyl]amino]-1-azetidinesulfonic acid, potassium salt

To a suspension of *cis*-3-benzyloxycarbonylamino-4-methylazetidinone (0.75 g) in 7 ml each of dimethylformamide (dried with 4A sieves activated at 320°C for 15 hours under argon flow) and methylene chloride (dried through basic $Al_2O_3$)

is added 1.66 g of pyridine-sulfur trioxide complex. After 3 hours stirring at room temperature under nitrogen, an additional amount of pyridine-sulfur trioxide complex (1.66 g) is added. The reaction mixture is then stirred at room temperature under nitrogen for about 16 hours. The dimethylformamide is removed in vacuo to give 4.6 g of residue which is dissolved in 300 ml of 0.5 M monobasic potassium phosphate solution (40°C for 10-15 minutes). The solution is cooled, passed through a column of HP-20 resin (3 cm x 60 cm) with 400 ml of 0.5 M monobasic potassium phosphate 1 L of distilled water and (14:1) water:acetone to give 280 mg of product in fractions 13 to 26 (100 ml each). Crystallization from MeOH: petroleum ether gives 757.5 mg of an analytical sample, melting point 214-215.5°C, dec.

Analysis calc'd for $C_{12}H_{13}N_2SO_6K$: C, 40.90; H, 3.72 N, 7.95; S, 9.10; K, 11.10

Found: C, 40.43; H, 3.60; N, 7.89; S, 8.69; K, 10.82

## Example 2

**(3S-trans)-4-Methyl-2-oxo-3-[[(phenylmethoxy)carbonyl]-amino]-1-azetidinesulfonic acid, potassium salt**

Following the procedure of Example 1, but substituting 1-t-boc-threonine for d,1-t-boc-allothreonine, yields the title compound, melting point 133-135°C.

Analysis calc'd for $C_{12}H_{13}N_2O_6SK$: C, 40.90; H, 3.72; N, 7.95; S, 9.10; K, 11.10

Found: C, 40.72; H, 3.60; N, 7.99; S, 8.80; K, 10.82

Example 3

(3S-trans)-4-Methyl-2-oxo-3-[(phenylacetyl)amino]-1-azetidinesulfonic acid, potassium salt

A) (3S)-3-Amino-4-methyl-2-oxo-1-azetidinesulfonic acid, tetrabutylammonium salt

(4S-trans)-4-Methyl-2-oxo-3-[[(phenylmethoxy)-carbonyl]amino]-1-azetidinesulfonic acid, potassium salt (352.4 mg; see Example 99) is dissolved in 20 ml of distilled water and treated with 373.5 mg (1 mmole) of tetrabutyl ammonium hydrogen sulfate. After 10 minutes stirring at room temperature, the solution is extracted three times with 10 ml portions of methylene chloride after saturation with sodium chloride. The methylene chloride is dried over sodium sulfate and evaporated in vacuo to give 536 mg of the tetrabutylammonium salt which is hydrogenated with 270 mg of 10% palladium on charcoal in 25 ml of dimethylformamide. The mixture is filtered through Celite and washed twice with 2.5 ml portions of dimethylformamide to yield the title compound in solution.

B) (3S-trans)-4-Methyl-2-oxo-3-[(phenylacetyl)-amino]-1-azetidinesulfonic acid, potassium salt

The crude (3S)-3-amino-4-methyl-2-oxo-1-azetidinesulfonic acid, tetrabutylammonium salt from part A (the combined filtrate and washings) are treated at 0°C with 206 mg of dicyclohexylcarbodiimide, 153 mg of N-hydroxybenzotriazole, and 138 mg of phenylacetic acid. The reaction

mixture is stirred at 0°C for 1 hour and then at room temperature for 2 hours. The resulting precipitate is filtered, the filtrate is evaporated in vacuo, the residue is dissolved in 10 ml of acetone and filtered. The filtrate is treated with 25 ml of acetone saturated with potassium iodide and then 200 ml of ether. The resulting solid (752.7 mg) is a mixture of the potassium and tetrabutylammonium salts of the title compound. The solid is dissolved in 50 ml of 0.5 monobasic potassium phosphate and applied to an HP-20 column. Elution with water and then water-acetone gives several fractions that are combined and evaporated to give the purified tetrabutylammonium salt. An aqueous solution of this material is passed through Dowex 50W-X2 ($K^+$form) to give the title potassium salt (121.4 mg). Trituration with acetone-hexane yields 104.6 mg of the title compound, melting point 211-213°C.

Analysis calc'd from $C_{12}H_{13}N_2O_5SK \cdot 1/2\ H_2O$: C, 41.72; H, 4.09; N, 8.11; S, 9.28; K, 11.32

Found: C, 41.70; H, 4.01; N, 8.07; S, 9.01; K, 11.02

## Example 4

(cis)-4-Methyl-2-oxo-3-[(phenylacetyl)amino]-1-azetidinesulfonic acid, potassium salt

A solution of 320 mg of (±—cis)-4-methyl-2-oxo-3-[[(phenylmethoxy)carbonyl]amino]-1- azetidinesulfonic acid, potassium salt (see example 98) is prepared in 20 ml of water containing 483 mg of tetrabutylammonium hydrogen sulfate and adjusted to pH 5.5.

Extraction with six 25 ml portions of methylene chloride gives 517.3 mg of oil. A solution of this material in 15 ml of dimethylformamide is stirred with 400 mg of 10% palladium on charcoal in an atmosphere of hydrogen for 90 minutes. The catalyst is filtered and the filtrate stirred with 150 mg of phenylacetic acid, 169 mg of N-hydroxybenzotriazole and 247 mg of dicyclohexylcarbodiimide for 7.5 hours. The solvent is removed in vacuo and the residue is dissolved in 20 ml of acetone and filtered. The filtrate is treated with 25 ml of 0.044 M potassium iodide in acetone. Dilution with an equal volume of ether gives a solid (330 mg) which is applied to a 50 ml HP-20 column in 20 ml of 0.05 M monobasic potassium phosphate. Elution with 200 ml of water followed by 1:9 acetone-water gives Rydon positive material in fractions (50 ml) 6-10. Evaporation of fractions 7-9 gives 81 mg of solid. Recrystallization from acetonitrile-water gives 46 mg of the title compound, which decomposes at >205°C. A second crop (6 mg) is obtained from the filtrate. A further 5 mg is obtained from fractions 6 and 10 by evaporation and recrystallization.

Analysis calc'd for $C_{12}H_{13}N_2O_5SK$: C, 42.84; H, 3.89 N, 8.33; S, 9.53; K, 11.62

Found: C, 42.75; H, 3.82; N, 8.32; S, 9.26 K, 11.63

Example 5

[3S-[3α(Z),4β]]-3-[[(2-Amino-4-thiazolyl)(methoxyimino)-acetyl]amino]-4-methyl-2-oxo-1-azetidinesulfonic acid, potassium salt

A) (3S-trans)-4-Methyl-2-oxo-3-[[(phenylmethoxy)-carbonyl]amino]-1-azetidinesulfonic acid, tetrabutylammonium salt

(3S-trans)-4-Methyl-2-oxo-3-[[(phenylmethoxy)-carbonyl]amino]-1-azetidinesulfonic acid, potassium salt (352.4 mg; see Example 99) is dissolved in 20 ml of water and tetrabutylammonium hydrogen sulfate (373.5 mg) is added. The aqueous solution is extracted three times with methylene chloride and the combined extracts are dried over sodium sulfate. After removal of the solvent, 534.6 mg of the title compound is obtained.

B) [3S-[3α(Z),4β]]-3-[[(2-Amino-4-thiazolyl)(methoxy-imino)acetyl]amino]-4-methyl-2-oxo-1-azetidinesulfonic acid, potassium salt

A solution of 534.6 mg of (3S-trans)-4-methyl-2-oxo-3-[[(phenylmethoxy)carbonyl]amino]-1-azetidine-sulfonic acid, tetrabutylammonium salt in 20 ml of dimethylformamide is hydrogenated with 220 mg of 10% palladium on charcoal at atmospheric pressure for 2.75 hours; the uptake of hydrogen is 26.3 ml. The mixture is filtered and washed twice with 2.5 ml of dimethylformamide. The filtrate and washings (total ca. 25 ml) are stirred under nitrogen with 161 mg of (Z)-α-(methoxyimino)-2-amino-4-thiazoleacetic acid, 136 mg of N-hydroxybenzotriazole

and 164.8 mg of dicyclohexylcarbodiimide. The mixture is stirred under nitrogen for about 16 hours. The dimethylformamide is removed in vacuo and the gummy residue is dissolved in acetone and filtered to remove urea. To the filtrate is added a solution containing 272 mg (0.8 mmole) of perfluorobutanesulfonic acid, potassium salt in 0.8 ml of acetone. The slurry is diluted with an equal volume of ether and filtered to give 325.5 mg of crude product which is purified through chromatography on 75 ml of HP-20AG. Elution with 400 ml of water and 400 ml of (9:1) water: acetone mixture (50 ml fractions) gives 335 mg in fractions 3 to 10. After trituration with acetone-hexane, 97.3 mg of an analytical sample is obtained from fractions 3-5. Similar trituration of fractions 6-10 gives an additional 90.4 mg of product as a solid.

Analysis calc'd for $C_{10}H_{12}N_5O_6S_2K$: C, 29.92; H, 3.01; N, 17.45; S, 15.97; K, 9.74

Found: C, 30.32; H, 3.49; N, 15.82; S, 13.95; K, 10.45

NMR(D20) 1.57 (3H, d, J=7), 3.97 (3H,S), 4.30(1H, d of q, J=7.3), 4.70(1H, d. J=7), 6.95ppm(1H,S).

## Example 6

**[3S-[3α(Z),4β]]-3-[[(2-Amino-4-thiazolyl)[(1-carboxy-1-methylethoxy)imino]acetyl]amino]-4-methyl-2-oxo-1-azetidinesulfonic acid, dipotassium salt**

A) N-Benzyloxy-t-boc-threonine amide

A solution of 8.76 g of t-boc-threonine and the free amine from 6.4 g of O-benzylhydroxylamine HCl (ethyl acetate-sodium bicarbonate liberation) in 100 ml of tetrahydrofuran is treated with 6.12 g

of N-hydroxybenzotriazole and 8.24 g of dicyclohexylcarbodiimide in 20 ml of tetrahydrofuran. The mixture is stirred under nitrogen for 26 hours, filtered, and evaporated *in vacuo*. The residue is chromatographed on a 300 g silica gel column (elution with chloroform and chloroform-ethyl acetate (3:1)) yielding 7.2 g of compound. Crystallization from ether-hexane gives 4.18 g of the title compound.

B) (3S-*trans*)-N-Benzyloxy-3-*t*-butoxycarbonylamino-4-methylazetidinone

A solution of 12.67 g of N-benzyloxy-*t*-boc-threonine amide, 11.5 g of triphenylphosphine, and 6.23 ml of diethylazodicarboxylate in 380 ml of tetrahydrofuran is stirred under nitrogen for about 16 hours. The solution is evaporated and chromatographed on a 900 gram silica gel column. Elution with chloroform-ethyl acetate (3:1) gives 13.69 g of compound that crystallizes from ether-hexane to yield 9.18 g of the title compound.

C) (3S-trans)-3-*t*-Butoxycarbonylamino-1-hydroxy-4-methylazetidinone

A solution of 9.18 g of (3S-*trans*)-N-benzyloxy-3-*t*-butoxycarbonylamino-4-methylazetidinone in 300 ml of 95% ethanol is stirred in an atmosphere of hydrogen with 1.85 g of 10% palladium on charcoal. After 141 minutes the slurry is filtered and evaporated *in vacuo*. The residue is recrystallized from ether-hexane to yield 5.12 g of the title compound.

D) (3S-trans)-3-t-Butoxycarbonylamino-4-methyl-azetidinone

A solution of 4.98 of (3S-trans)-3-t-butoxy-carbonylamino-1-hydroxy-4-methylazetidinone in 200 ml of methanol is treated with 132 ml of 4.5 M ammonium acetate and then 66 ml of 1.5 M titanium trichloride and stirred for 4.5 hours. The aqueous solution is diluted with an equal volume of 8% sodium chloride and extracted with ethyl acetate to give 3.48 g of crude product. Recrystallization from ether-hexane yields 3.3 g of the title compound.

E) (3S-trans)-3-Benzyloxycarbonylamino-4-methyl-azetidinone

A solution of 3.3 g of (3S-trans)-3-t-butoxycarbonylamino-4-methylazetidinone in 10 ml each of dichloromethane and anisole is cooled to $0^{\circ}C$ and 112 ml of trifluoroacetic acid is added. The solution is stirred for 90 minutes and evaporated *in vacuo* (benzene added and evaporated three times). The residue is dissolved in 70 ml of acetone and the solution is adjusted to pH 7 with 5% sodium bicarbonate solution. A total of 5.33 g of benzyl chloroformate is added over 1 hour at pH 6.5-7.5. The mixture is stirred for 30 minutes at pH 7, diluted with 100 ml of saturated salt, and extracted with ethyl acetate (three 400 ml portions). The residue obtained by evaporation is chromatographed on a 1 liter silica gel column. Elution with chloroform-ethyl acetate (4:1) gives 2.19 g of compound. Crystallization from ether-hexane yields 1.125 g of the title compound.

F) (3S-*trans*)-4-Methyl-2-oxo-3-[[(phenylmethoxy)-carbonyl]amino]-1-azetidinesulfonic acid, tetrabutylammonium salt

A solution of 600 mg of (3S-*trans*)-3-benzyloxycarbonylamino-4-methylazetidinone in 2 ml of dimethylformamide is cooled to 0°C and 4 ml of 0.8 M sulfur trioxide in dimethylformamide is added. The solution is stirred at room temperature under nitrogen for 1 hour and poured into 80 ml of cold 0.5 M monobasic potassium phosphate (adjusted to pH 5.5). The solution is extracted with three 50 ml portions of methylene chloride (discarded) and 868 mg of tetrabutylammonium bisulfate is added. The resulting solution is extracted with four 75 ml portions of methylene chloride. The combined organic layer is washed with 8% aqueous sodium chloride, dried, and evaporated *in vacuo* yielding 1.54 g of the title compound.

G) [3S-[3α(Z),4β]]-3-[[(2-Amino-4-thiazolyl)-[(1-diphenylmethoxycarbonyl-1-methylethoxy)imino]-acetyl]amino]-4-methyl-2-oxo-1-azetidinesulfonic acid, potassium salt

A solution of 1.54 g of (3S-*trans*)-4-methyl-2-oxo-3-[[(phenylmethoxy)carbonyl]amino]-1-azetidinesulfonic acid, tetrabutylammonium salt in 45 ml of dimethylformamide is stirred in an atmosphere of hydrogen with 800 mg of 10% palladium on charcoal for 2 hours. The catalyst is filtered and the filtrate stirred for about 16 hours with

1.24 g of (Z)-2-amino-α-[(1-diphenylmethoxycarbonyl-1-methylethoxy)imino]-4-thiazoleacetic acid, 0.4 g of N-hydroxybenzotriazole, and 580 mg of dicyclohexylcarbodiimide. The slurry is evaporated in vacuo and the residue is triturated with 20 ml of acetone and filtered. The filtrate (plus 2 ml of washings) is treated with 868 mg of potassium perfluorobutanesulfonate in 3 ml of acetone. Dilution with 75 ml of ether gives a solid that is isolated by decantation of the mother liquor, trituration with ether, and filtration to give 0.91 g of the title compound. The mother liquor is diluted with a further 100 ml of ether to give a second crop, 0.45 g, of the title compound.

H) [3S-[3α(Z),4β]]-3-[[(2-Amino-4-thiazolyl)[(1-carboxy-1-methylethoxy)imino]-acetyl]amino]-4-methyl-2-oxo-1-azetidinesulfonic acid, dipotassium salt

A slurry of 140 mg of [3S-[3α(Z),4β]]-3-[[(2-amino-4-thiazolyl)[(1-diphenylmethoxycarbonyl-1-methylethoxy)imino]acetyl]amino]-4-methyl-2-oxo-1-azetidinesulfonic acid, potassium salt (first crop) in 0.5 ml of anisole is stirred at -12°C under nitrogen and 2.5 ml of cold (-10°C) trifluoroacetic acid is added. After 10 minutes, 10 ml of ether and 5 ml of hexane are added and the resulting slurry is stirred for 5 minutes at -12°C, and allowed to warm to room temperature. The solid is isolated by centrifugation and washed twice with ether. A solution of this solid in 5 ml of cold water is

immediately adjusted to pH 5.5 with 0.4 N potassium hydroxide and then applied to an 80 ml HP-20AG column. Elution with water gives 72 mg of the title compound in fractions (10 ml) 7-11 after evaporation (acetonitrile added and evaporated three times) and trituration with ether, m.p. ~250°(dec.).

Analysis calc'd for $C_{13}H_{15}N_5O_8S_2K_2$: C, 30.51; H, 2.95; N, 13.69; S, 12.53; K, 15.28

Found: C, 29.63; H, 3.20; N, 12.96; S, 11.94; K, 12.78

NMR($D_2O$) 1.46(S, 6H), 1.58(1H, d, J=7) 4.28(1H, d of q, J=7, 2.5), 4.67 (1H, d, J=2), 6.95ppm(S, 1H).

The remaining 1.22 g of [3S-[3α(Z),4β]]-3-[[(2-amino-4-thiazolyl)[(1-diphenylmethoxycarbonyl-1-methylethoxy)imino]acetyl]amino-4-methyl-2-oxo-1-azetidinesulfonic acid, potassium salt (crops 1 and 2) are treated as above (4.2 ml anisole, 16 ml of trifluoroacetic acid, 13 minutes at -15°C). Chromatography on a 300 ml HP-20AG column gives 694 mg of the title compound in fractions (60 ml) 6-9 after treatment as above.

Example 7

[3α(Z),4α]-3-[[(2-Amino-4-thiazolyl)(methoxyimino)acetyl]amino]-4-methyl-2-oxo-1-azetidinesulfonic acid, potassium salt

A solution of 51.8 mg of (cis)-4-methyl-2-oxo-3-[[(phenylmethoxy)carbonyl]amino]-1-azetidinesulfonic acid, potassium salt and 51 mg of tetra-n-butyl-ammonium bisulfate in 5 ml of water is extracted with methylene chloride (four 10 ml portions) to give 81 mg of oil. This is stirred in an atmosphere of hydrogen for 2 hours with 40 mg of 10% palladium on charcoal in 4 ml of dimethylformamide. The catalyst is filtered and washed with 1 ml of dimethylformamide. The filtrate and washings are combined and stirred for about 16 hours with 31 mg of (Z)-2-amino-α-(methoxyimino)-4-thiazoleacetic acid, 27 mg of N-hydroxybenzotriazole, and 31.5 mg of dicyclohexylcarbodiimide. The solution is evaporated in vacuo and the residue triturated with 3 ml of acetone. The resulting slurry is centrifuged and the liquid treated with 51 mg of potassium perfluorobutanesulfonate. Dilution with 5 ml of ether and filtration gives a solid. Chromatography on HP-20 AG (40 ml) gives Rydon positive material in fractions (20 ml) 3-5 (elution with water). Evaporation and ether trituration gives 23 mg of product, as a hygroscopic solid.

Analysis calc'd for $C_{10}H_{12}N_5O_6S_2K$:

| | |
|---|---|
| | C,29.91; H,3.01; N,17.44 |
| Found: | C,29.30; H,3.31; H,16.66 |

NMR($D_2O$) 1.40(3H, d, J=7), 3.97 (3H,S), 4.46 (1H, apparent pentet, J=7), 5.37(1H, d, J=7), 6.97 ppm(1H,S).

## Example 8

## (3S-*cis*)-3-Amino-4-methyl-2-oxo-1-azetidinesulfonic acid

### A) *t*-Boc-l-allothreonine

A suspension of 6.72 g of l-allothreonine in 70 ml of 50% aqueous dioxane is treated with 9.45 ml of triethylamine and 18.1 g of t-butylpyrocarbonate. The resulting mixture is stirred at room temperature for 4 hours, and then diluted with 70 ml of water and 140 ml of ethyl acetate. After thorough shaking, the layers are separated and the organic layer is washed with 30 ml of 2:1 water:brine. Combined aqueous layers are then back-extracted with 70 ml of ethyl acetate. The aqueous layer is cooled in an ice bath and 10% potassium bisulfite solution is added to pH 2.3. The acidified solution is extracted with ethyl acetate (four 150 ml portions). Combined organic layers are dried over anhydrous sodium sulfate and stripped of solvent to give 9.13 g of the title compound.

### B) N-Methoxy-*t*-boc-l-allothreonine amide

*t*-Boc-l-allothreonine (9.13 g) is dissolved in 85 ml of water and 41 ml of 1N potassium hydroxide solution. Methoxyamine hydrochloride (5.22 g) and 8.67 g of 1-ethyl-3,3-(dimethyl-aminopropyl)carbodiimide·HCl are added. The

mixture is stirred at room temperature for 4 hours and then saturated with sodium potassium tartarate. The resulting mixture is extracted with ethyl acetate (four 150 ml portions) and the organic layer is dried over anhydrous sodium sulfate and stripped of solvent to give 7.38 g of the title compound as a solid.

C) O-Methanesulfonyl-N-methoxy-t-boc-l-allothreonine amide

N-Methoxy-t-boc-l-allothreonine amide (7.32 g) is dissolved in 40 ml of pyridine and cooled to -20°C under nitrogen. Methanesulfonyl chloride (3 ml) is added dropwise by syringe over a 5-minute period. The resulting mixture is slowly warmed to 0°C and stirred at that temperature for 3 hours. Ethyl acetate (500 ml) is added and the solution washed with 250 ml of ice-cold 3N HCl solution, then 100 ml of 5% $NaHCO_3$ solution. The ethyl acetate layer was dried over anhydrous sodium sulfate and stripped of solvent to give 8.64 g of the title compound as a white solid.

D) (3S-cis)-3-t-Butoxycarbonylamino-1-methoxy-4-methylazetidinone

O-Methanesulfonyl-N-methoxy-t-boc-l-allothreonine amide (8.64 g) is dissolved in 530 ml of acetone and 11 g of solid potassium carbonate is added. The mixture is slowly heated to 65°C under nitrogen and stirred at this temperature for one hour. The reaction mixture is then

filtered through Celite and the filter cake is washed with ethyl acetate. The filtrate is concentrated and the residue is taken up in 250 ml of ethyl acetate. The ethyl acetate solution is washed with 100 ml of 1N hydrochloric acid solution and 100 ml of 5% sodium bicarbonate solution. The ethyl acetate layer is dried over anhydrous sodium sulfate and stripped of solvent to give 6.63 g of crude product.

E) (3S-*cis*)-3-*t*-Butoxycarbonylamino-4-methyl-azetidinone

Sodium (1.35 g) is dissolved in about 300 ml of liquid ammonia at -50°C and 5.87 g of (3S-*cis*)-3-*t*-Butoxycarbonylamino-1-methoxy-4-methylazetidinone in 35 ml tetrahydrofuran is added dropwise via syringe. An additional 10 ml of tetrahydrofuran is used for rinsing. Near the end of the addition about 100 mg of additional sodium is added. The mixture is stirred for five more minutes, then quenched by adding 3.35 g of solid ammonium chloride in one portion. Ammonia is blown off with a nitrogen stream and 250 ml of ethyl acetate is added to the residue. After filtration and washing the solid with ethyl acetate, the combined filtrate is stripped of solvent to give 4.82 g of the title compound.

F) (3S-*cis*)-3-*t*-Butoxycarbonylamino-4-methyl-2-oxo-1-azetidinesulfonic acid, tetrabutyl ammonium salt

[3S,4R]-3-*t*-Butoxycarbonylamino-4-methyl-azetidinone (4.98 g) is dissolved in 30 ml of dimethylformamide. Pyridine-sulfur trioxide complex 11.9 g is added and the mixture is stirred at room temperature under nitrogen. After 14 hours stirring, an additional 1.8 g of pyridine-sulfur trioxide complex is added and stirring is continued for 80 hours. The reaction mixture is poured into 700 ml of 0.5 M monobasic potassium phosphate solution and washed with methylene chloride (three 300 ml portions). tetra-n-Butylammonium bisulfate (8.45 g) is added to the aqueous solution and the mixture is extracted with methylene chloride (four 300 ml portions). The combined methylene chloride layers are dried over anhydrous sodium sulfate and stripped of solvent to give 10.76 g of the title compound as a gum.

G) (3S-*cis*)-3-Amino-4-methyl-2-oxo-1-azetidine-sulfonic acid

(3S-*cis*)-3-*t*-Butoxycarbonylamino-4-methyl-2-oxo-1-azetidinesulfonic acid, tetrabutyl ammonium salt (10.76 g) is dissolved in 50 ml of 95-97% formic acid and stirred for 4 hours under nitrogen. A small amount of product from a previous reaction is added as seed and the mixture is stirred for one more hour. The

mixture is stored in the freezer for about 16 hours and the frozen mixture is warmed to room temperature and stirred for an additional hour. The solid formed is filtered and washed with methylene chloride to yield 982 mg of the title compound. The filtrate is diluted with 1 liter of methylene chloride and kept at $-20^{o}C$ for 4 hours. The precipitate that forms is recrystallized from water-methanol-acetone to give an additional 167 mg of title compound.

NMR($D_{2}0$) 1.63 (3H, d, $J$=6.5 cps), IR(nujol)1775 $cm^{-1}$.

Example 9

[3S-[3α(Z),4α]]-3-[[2-Amino-4-thiazolyl)-methoxyimino)acetyl]amino-4-methyl-2-oxo-1-azetidinesulfonic acid, potassium salt

A solution of 201 mg of (Z)-2-amino-α-(methoxyimino)-4-thiazoleacetic acid and 153 mg of N-hydroxy-benzotriazole monohydrate in 3 ml of dimethylformamide is treated with 206 mg of dicyclohexylcarbodiimide. The mixture is stirred at room temperature for 20 minutes under nitrogen and a solution of 180 mg of (3S-cis)-3-amino-4-methyl-2-oxo-1-azetidinesulfonic acid and 0.14 ml of triethylamine in 2 ml of dimethylformamide is added (an additional 1 ml of dimethylformamide is used for rinsing) and the mixture is stirred for about 16 hours. The slurry is evaporated in vacuo, triturated with 12 ml of acetone, centrifuged and the liquid treated with 338 mg of potassium perfluorobutanesulfonate. Dilution with 10 ml of ether and filtration gives a solid product which is chromatographed on 200 ml of HP-20 resin eluting with

water. Fractions (20 ml each) 18-30 are combined and lyophilized to give 274 mg of the title compound as a hygroscopic solid.

Anal. calc'd. for $C_{10}H_{12}N_5O_6S_2K$: C,29.91; H, 3.01; N,17.44

Found: C,30.03; H,3.21; N,17.06

NMR($D_{2}0$) 1.40 (3H, d, J=6.5), 3.98(3H,S), 4.48 1H, d of t, J=6.4, 5.5), 5.36(1H, d, J=5.5) 6.97 (1H,S).

## Example 10

(3S-trans)-3-Amino-4-methyl-2-oxo-1-azetidine-sulfonic acid

A) Threonine, methyl ester, hydrochloride

Under an atmosphere of nitrogen, a flask containing 500 ml of methanol is cooled to -5°C (ice/brine) and 130 ml (excess) of thionyl chloride is added at such a rate as to maintain the reaction temperature between 0° and 10°C. After recooling to -5°C, 59.5 g of l-threonine is added and the mixture is allowed to reach room temperature and stirred for 16 hours. The mixture is concentrated and evacuated at $10^{-1}$ torr for 2 hours to yield a viscous oil. This material is used directly in the following step.

B) Threonine amide

The crude product from part A is dissolved in 2.5 l of methanol and cooled to -5°C (ice/brine). The solution is saturated with ammonia gas, the cooling bath is removed and the sealed vessel is allowed to stand for 3 days. After removing the bulk of the unreacted ammonia via aspirator, 100 g of sodium bicarbonate and 50 ml of water is added and the mixture is stripped to a viscous oil.

C) Benzyloxycarbonylthreonine amide

The crude product from part B (already containing the requisite amount of sodium bicarbonate is diluted to a volume of 1 liter with water. To this rapidly stirring solution, 94 g (88 ml of 90% pure material) of benzyloxycarbonyl chloride is added as a solution in 80 ml of tetrahydrofuran over a 1 hour period. The reaction mixture is then stirred for an additional 16 hours and extracted with ethyl acetate (one 500 ml portion, two 250 ml portions). The combined extracts are dried over magnesium sulfate and concentrated. The crystalline residue is then dissolved in 250 ml of hot ethyl acetate and 300 ml of hexane is added followed by boiling until a clear solution is reached. Cooling and filtration of the crystalline mass give, after drying, 104 g of the title compound.

D) Benzyloxycarbonylthreonine amide, O-mesylate

Under an atmosphere of argon, 100 g of benzyloxycarbonylthreonine amide is dissolved in 400 ml of anhydrous pyridine and cooled in an ice/salt bath. To this stirring solution, 36.8 ml (54.5 g) of methanesulfonyl chloride is added over a 15 minute period. After 2 hours of stirring an additional 0.3 equivalents of methanesulfonyl chloride is added. The reaction is then stirred for 1 hour and poured into a mixture of 1.5 l of ice and 2 l of water. The resulting slurry is stirred for about 30 minutes and filtered. Drying of the crude product at 60°C for about 16 hours in a vacuum oven gives 109 g of the title compound.

E) N-Sulfonyl benzyloxycarbonylthreonine amide, O-mesylate, tetrabutylammonium salt

A solution of 2-picoline (17.8 ml) in 90 ml of methylene chloride is cooled to -5°C (ice-brine) and chlorosulfonic acid 5.97 ml is added at such a rate as to maintain the internal reaction temperature below 5°C. The resulting solution is added via canula, to a suspension of 7.56 g of benzyloxycarbonylthreonine amide, O-mesylate in 120 ml of methylene chloride. The resulting heterogeneous mixture is refluxed for about 16 hours yielding a clear solution. The solution is poured into 500 ml of pH 4.5 phosphate buffer (0.5 M) and further diluted with 120 ml of methylene chloride. The separated

organic layer is then washed once with 100 ml of buffer solution and the combined aqueous phases are treated with 10.2 g of tetra-n-butylammonium hydrogensulfate and extracted with methylene chloride (one 300 ml portion and two 150 ml portions). After drying the combined organic extracts over sodium sulfate, the solution is concentrated to yield 12.7 g of a foam.

F) (3S-*trans*)-3-Amino-4-methyl-2-oxo-1-azetidinesulfonic acid

A mixture consisting of 5.52 g of potassium carbonate in 20 ml of water and 160 ml of 1,2-dichloroethane is brought to reflux and 15.5 mmole of N-sulfonyl benzyloxycarbonylthreonine amide, O-mesylate, tetrabutylammonium salt is added in 20 ml of 1,2-dichloroethane (20 ml used as a rinse). After refluxing for 30 minutes, the mixture is poured into a separatory funnel, diluted with 50 ml of water and 100 ml of methylene chloride and the phases split. The resulting organic phase is dried over sodium sulfate and concentrated to yield crude (3S-*trans*)-3-benzyloxycarbonylamino-4-methyl-2-oxo-1-azetidinesulfonic acid, tetrabutylammonium salt. The crude azetidinone is treated in 250 ml of ethanol with 0.8 g of 5% palladium on charcoal catalyst and hydrogen is bubbled through the solution. After 90 minutes the mixture is filtered through Celite with 50 ml of ethanol used as a rinse.

The addition of 1.2 ml of formic acid to this solution causes an immediate precipitation of the title zwitterion which is filtered after stirring for 1 hour to yield, after drying at $10^{-1}$ torr for 1 hour, 1.1 g of product. A second crop of product is obtained upon concentration of the filtrate and addition of more formic acid to give 1.3 g of the title zwitterion. m.p. >218°dec., $[\alpha]_D = -41.1 (C=1, H_2O)$. NMR($D_{2}0$) 1.58(3H, d J=7), 4.80(2H,M).

Examples 11-14

Following the procedure of Example 9, but substituting (3S-trans)-3-amino-4-methyl-2-oxo-1-azetidinesulfonic acid for (3S-cis)-3-amino-4-methyl-2-oxo-1-azetidinesulfonic acid and the acid listed in column I for (Z)-2-amino-α-(methoxyimino)-4-thiazoleacetic acid, yields the compound listed in column II.

| | |
|---|---|
| 11. (R)-α-[[[3-[(2-furanylmethylene)amino]-2-oxo-1-imidazolidinyl]-carbonyl]amino]-benzeneacetic acid | [3S-[3α(R*),4β]]-3-[[[[[3-[(2-furanyl-methylene)amino]-2-oxo-1-imidazolidinyl]-carbonyl]amino]phenylacetyl]amino]-4-methyl-2-oxo-1-azetidinesulfonic acid, potassium salt; melting point 213°C, dec. |
| 12. (R)-α-[[(4-ethyl-2,3-dioxo-1-piperazinyl)-carbonyl]amino]benzeneacetic acid | [3S-[3α(R*),4β]]-3-[[[[(4-ethyl-2,3-dioxo-1-piperazinyl)carbonyl]amino]phenylacetyl]-amino]-4-methyl-2-oxo-1-azetidinesulfonic acid, potassium salt; melting point 177°C,dec. |
| 13. (Z)-2-amino-α-(hydroxyimino)-4-thiazole-acetic acid | [3S-[3α(Z),4β]]-3-[[(2-amino-4-thiazolyl)-(hydroxyimino)acetyl]amino]-4-methyl-2-oxo-1-azetidinesulfonic acid, potassium salt; melting point 230°C. |
| 14. (±)-α-[(aminooxoacetyl)amino]-2-thio-pheneacetic acid | [3S-[3α(±),4β]]-3-[[[(aminooxoacetyl)amino]-2-thienylacetyl]amino]-4-methyl-2-oxo-1-azetidine-sulfonic acid, potassium salt; melting point 135 °C, dec. |

Example 15

[3S-[3α(Z),4β]]-3-[[(2-Amino-4-thiazolyl)[[1,1-dimethyl-2-[(4-nitrophenyl)methoxy]-2-oxoethoxy]-imino]acetyl]amino]-4-methyl-2-oxo-1-azetidine-sulfonic acid, potassium salt

To a slurry of (3S-trans)-3-amino-4-methyl-2-oxo-1-azetidinesulfonic acid (0.36 g; see example 139) in dry dimethylformamide (30 ml) under nitrogen at 26°C is added triethylamine (309 μl). After about 5 minutes a clear solution is obtained and (Z)-2-amino-α-[[1,1-dimethyl-2-[(4-nitrophenyl)methoxy]-2-oxoethoxy]-imino]-4-thiazoleacetic acid (0.816 g) is added followed by N-hydroxybenzotriazole (0.334 g) and dicyclohexylcarbodiimide (0.453 g). The mixture is stirred for twelve hours at 26°C, whereupon the solvent is removed in vacuo, and the residue is triturated with acetone (30 ml). After stirring five minutes, the solids are removed and the filtrate is treated with potassium perfluorobutane sulfonate (3.680 g) in acetone, (5 ml). Addition of ether (approximately 40 ml) affords a precipitate which is collected and dried in vacuo (1.073 g; second crop 0.066 g; total of 1.14 g).

Anal. calc'd. for $C_{20}H_{21}N_6O_{10}S_2K \cdot 1\ H_2O$: C, 38.33; H, 3.70; N, 13.41; S, 10.23; K, 6.24

Found: C, 38.30; H, 3.63; N, 13.41; S, 9.88; K, 5.98

Example 16

[3α(Z),4α]-3-[[(2-Amino-4-thiazolyl)[(1-carboxy-1-methylethoxy)imino]acetyl]amino]-4-methyl-2-oxo-1-azetidinesulfonic acid, potassium salt (1:2)

A) [3α(Z),4α]-3-[[2-Amino-4-thiazolyl)[(1-diphenylmethoxycarbonyl-1-methylethoxy)imino]-acetyl]amino]-4-methyl-2-oxo-1-azetidinesulfonic acid, potassium salt

A solution of (cis)-4-methyl-2-oxo-3-[[(phenylmethoxy)carbonyl]amino]-1-azetidinesulfonic acid, tetrabutylammonium salt (201 mg.; prepared from the corresponding potassium salt of example 98 as described in example 136) in 5 ml of dimethylformamide is stirred with 90 mg of 10% palladium on calcium carbonate in an atmosphere of hydrogen for 2 hours. The slurry is filtered and the filtrate is stirred for about 16 hours with 146 mg of (Z)-2-amino-α-[1-diphenylmethoxycarbonyl-1-methylethoxy)imino]-4-thiazoleacetic acid, 73 mg of dicyclohexylcarbodiimide and 51 mg of N-hydroxybenzotriazole under nitrogen. The slurry is evaporated *in vacuo* and triturated with 4 ml of acetone. The slurry is filtered and the solid washed twice with 2 ml portions acetone. The filtrate and washings are combined and treated with 113 mg of potassium perfluorobutanesulfonate. Dilution with 24 ml of ether gives a solid that is isolated by centrifugation and washed three times with ether yielding 186 mg of the title compound.

B) <u>[3α(Z),4α]-3-[[(2-Amino-4-thiazolyl)[1-carboxy-1-methylethoxy]imino]acetyl]amino]-2-methyl-4-oxo-1-azetidinesulfonic acid, potassium salt (1:2)</u>

A slurry of 186 mg of [3α(Z),4α]-3-[[2-amino-4-thiazolyl)[(1-diphenylmethoxycarbonyl-1-methylethoxy)imino]acetyl]amino]-4-methyl-2-oxo-1-azetidinesulfonic acid, potassium salt in 0.6 ml of distilled anisole is cooled to -12°C and 3.0 ml of distilled trifluoroacetic acid (at -10°C) is added. The solution is stirred for 10 minutes and 12 ml of ether followed by 6 ml of hexane are added. After 5 minutes at -10°C and stirring for 15 minutes at ambient temperature, the solid is isolated by centrifugation and washed four times with ether to give 141 mg of material. This is dried *in vacuo*, powdered, dissolved in 5 ml of cold water and immediately adjusted to pH 5.6 with 0.4 N potassium hydroxide. The solution is applied to a 100 ml HP-20AG column and eluted with water. Fractions (10 ml) 8-12 are combined and evaporated *in vacuo* (acetonitrile is added three times and evaporated. The residue is triturated with ether to give 101.7 mg of product, as a hygroscopic solid.

<u>Anal</u>. <u>Calc'd</u> for $C_{13}N_{15}N_5O_8S_2\cdot 2K$: C, 30.51; H, 2.95; N, 13.69; S, 12.53; K, 15.28

Found: C, 30.11; H, 3.26; N, 13.35; S, 12.12; K, 15.02

Example 17

[3S-[3α(Z),4β]]-3-[[(2-Amino-4-thiazolyl)-[(1-carboxy-1-methylethoxy)imino]acetyl]amino]-4-methyl-2-oxo-1-azetidinesulfonic acid

[3S-[3α(Z),4β]]-3-[[(2-Amino-4-thiazolyl)-[(1-carboxy-1-methylethoxy)imino]acetyl]amino]-4-methyl-2-oxo-1-azetidinesulfonic acid, dipotassium salt (87.3 mg; see example 103) is dissolved in 1.38 ml of water, cooled to 0°C, treated with 0.34 ml of 1N hydrochloric acid and the resulting crystals separated by centrifugation. The wet solid is dissolved in methanol, filtered, concentrated to about 0.5 ml and mixed with 1 ml of water, giving 55.9 mg of the title compound.

Example 18

[3S-[3α(Z),4β]]-3-[[(2-Amino-4-thiazolyl)[(1-carboxy-1-methylethoxy)imino]-4-methyl-2-oxo-1-azetidinesulfonic acid, sodium salt

A 99.7 mg sample of [3S-[3α(Z),4β]]-3-[[(2-amino-4-thiazolyl)[(1-carboxy-1-methylethoxy)imino]acetyl]amino]-4-methyl-2-oxo-1-azetidinesulfonic acid is mixed with 0.207 ml of 1N sodium hydroxide and the resulting mixture is gently warmed to dissolve the remaining solid. Water is removed azeotropically with acetonitrile and the residue is crystallized from a mixture of 0.5 ml of methanol (to dissolve the residue) and 1 ml of acetonitrile, giving 81.8 mg of solid. A second recrystallization from 0.8 ml of methanol gives 47.9 mg, a third from 0.24 ml of methanol and 0.24 ml of absolute ethanol gives 44.8 mg, and

a fourth from 0.225 ml of methanol and 0.225 ml of absolute ethanol gives 38.8 mg. The solid is dried at 20°C and 0.01 mm of Hg for 18 hours and then equilibrated with atmospheric moisture for 24 hours, giving 40.9 mg of the title compound.

## Example 19

[3S-[3α(Z),4β]]-3-[[(2-Amino-4-thiazolyl)[(1-carboxy-1-methylethoxy)imino]-4-methyl-2-oxo-1-azetidinesulfonic acid, disodium salt

A 3.00 g sample of [3S-[3α(Z),4β]]-3-[[(2-Amino-4-thiazolyl)-[(1-carboxy-1-methylethoxy)imino]acetyl]amino]-4-methyl-2-oxo-1-azetidinesulfonic acid, (see example 146), is suspended in 30 ml of water and titrated with 1N sodium hydroxide requiring 12.0 ml to give the title disodium salt. The pH is reduced to 6.5 by the addition of a little Dowex 50W-X2 ($H^+$). The mixture is filtered and the filtrate diluted to 66.3 g with water. A 6.63-g portion is removed for other purposes. The remaining filtrate is lyophilized, giving 2.38 g of solid. Partial equilibration (24 hours) with atmosphereic moisture gives 2.54 g of the title compound.

## Examples 20-22

Following the procedure of Example 9, but substituting the compound listed in column I for (Z)-2-amino-α-(methoxyimino)-4-thiazoleacetic acid, yields the compound listed in column II.

| | Column I | Column II |
|---|---|---|
| 20. | (R)-α-[[[2,3-dioxo-4-[[(phenylmethoxy)-carbonyl]amino]-1-piperazinyl]carbonyl]-amino]benzeneacetic acid | [3S-[3α(R*),4α]]-[4-[[[2-[(4-methyl-2-oxo-1-sulfo-3-azetidinyl)amino]-2-oxo-1-phenylethyl]amino]carbonyl]-2,3-dioxo-1-piperazinyl]carbamic acid, phenylmethyl ester, potassium salt, melting point 191°C,dec. |
| 21. | (R)-α-[[[[(2-furanylmethylene)amino]-2-oxo-imidazolidinyl]carbonyl]amino]benzeneacetic acid | [3S-[3α(R*),4α]]-3-[[[[[3-[(2-furanylmethylene)-amino]-2-oxo-1-imidazolidinyl]carbonyl]-amino]phenylacetyl]amino]-4-methyl-2-oxo-1-azetidinesulfonic acid, potassium salt |
| 22. | (R)-α-[[(4-ethyl-2,3-dioxo-1-piperazinyl)-carbonyl]amino]benzeneacetic acid | [3S-[3α(R*),4α]]-3-[[[[(4-ethyl-2,3-dioxo-1-piperazinyl)carbonyl]amino]phenylacetyl]-amino]-4-methyl-2-oxo-1-azetidinesulfonic acid, potassium salt |

Example 23

[3S-[3α(Z),4α]]-3-[[(2-Amino-4-thiazolyl)[(1-carboxy-1-methylethoxy)imino]acetyl]amino]-4-methyl-2-oxo-1-azetidinesulfonic acid, potassium salt (1:2)

A) [3S-[3α(Z),4α]]-3-[[(2-Amino-4-thiazolyl)-[(1-diphenylmethoxycarbonyl-1-methylethoxy)imino]-acetyl]amino]-4-methyl-2-oxo-1-azetidinesulfonic acid, potassium salt

A solution of 440 mg of (Z)-2-amino-α-[(1-carboxy-1-methylethoxy)imino]-4-thiazoleacetic acid and 153 mg of N-hydroxybenzotriazole monohydrate in 3 ml of dimethylformamide is treated with 206 mg of dicyclohexylcarbodiimide. The mixture is stirred at room temperature for 30 minutes under nitrogen and a solution of 180 mg of (3S-cis)-3-amino-4-methyl-2-oxo-1-azetidinesulfonic acid, (see example 137) and 0.14 ml of triethylamine in 2 ml of dimethylformamide is added (an additional 1 ml of dimethylformamide is used for rinsing) and the mixture is stirred for about 16 hours. The slurry is evaporated *in vacuo* and triturated with 12 ml of acetone. The slurry is filtered and the solid washed with acetone (two 3 ml portions). The combined filtrate and washings are treated with 338 mg of potassium perfluorobutanesulfonate. Dilution with 30 ml of ether gives a gummy solid, which slowly solidifies. The solid is filtered and washed with ether to give 656 mg of the title compound.

B) <u>[3S-[3α(Z),4α]]-3-[[(2-Amino-4-thiazolyl)-[(1-carboxy-1-methylethoxy)imino]acetyl]amino]-4-methyl-2-oxo-1-azetidinesulfonic acid, potassium salt (1:2)</u>

A slurry of 656 mg of [3S-[3α(Z),4α]]-3-[[(2-Amino-4-thiazolyl)[(1-diphenylmethoxycarbonyl-1-methylethoxy)imino]acetyl]amino]-4-methyl-2-oxo-1-azetidinesulfonic acid, potassium salt in 2.3 ml of distilled anisole is cooled to -12°C and 11.5 ml of trifluoroacetic acid (precooled to -10°C) is added. The solution is stirred for 15 minutes and 46 ml of ether followed by 23 ml of hexane are added. After 5 minutes at -10°C and stirring for 15 minutes at room temperature, the solid is filtered and washed with ether to give 457 mg of a very hydroscopic gum. This is dissolved in 6 ml of cold water and immediately adjusted to pH 5.6 with 0.4N potassium hydroxide solution. The solution is applied to a 200 ml HP-20 resin and eluted with water. Fractions (50 ml each) 7-11 are combined and lyophilized to give 239 mg of the title compound as a solid.

<u>Anal.</u> <u>Calc'd.</u> for $C_{13}H_{15}O_8N_5S_2K_2 \cdot 1/2\ H_2O$: C, 29.99; H, 3.10; N, 13.45; S, 12.32

Found: C, 29.94; H, 3.30; N, 13.30; S, 11.93

NMR($D_{2}0$) 1.44 (3H d, J=75), 1.46(6H,S), 4.48(1H,d of t J=75, 5.5), 5.34(1H, d, J=5.5) 6.96ppm(1H,S).

Example 24

(±)-3-Amino-4,4-dimethyl-2-oxo-1-azetidinesulfonic acid

A) (±)-4,4-Dimethyl-2-oxo-1-azetidine-tertbutyldiphenylsilane

A solution of 40.5 ml of t-butylchlorodiphenylsilane in 112 ml of dimethylformamide is cooled to 0°C. To this is added 22 ml of triethylamine. A solution of 12.87 g of 4,4-dimethyl-2-azetidinone in 25 ml of dimethylformamide is added dropwise over 10 minutes to the cooled triethylamine solution. The resulting cloudy solution is stirred for 18 hours at 5°C under argon. This mixture is poured into 400 ml of ice water and extracted with three 150 ml portions of 2:1 ether:ethyl acetate. The combined extracts are washed with four 100 ml portions of 0.5M monobasic potassium phosphate buffer, one 150 ml portion of sodium bicarbonate solution, two 150 ml portions of water and one 150 ml portion of saturated sodium chloride solution. The solution is dried over sodium sulfate and concentrated *in vacuo* to yield 33.03 g of the title compound as a solid.

B) (±)-3-azido-4,4-dimethyl-2-oxo-1-azetidine-tertbutyldiphenylsilane

A solution of 4.25 ml of 1.6M (in hexane) n-butyllithium and 11ml of dry tetrahydrofuran is prepared at -50°C under argon in a 100 ml three-necked flask. A solution of 0.083 g of triphenylmethane in 1 ml of tetrahydrofuran is

added. The resulting solution is cooled to -60°C, and 1.0 ml of diisopropylamine is added dropwise by syringe. This is stirred for 15 minutes and then cooled to -78°C. A solution of 2.3 g of (±)-4,4-dimethyl-2-oxo-1-azetidine-tertbutyldiphenylsilane in 8 ml of tetrahydrofuran is added slowly by syringe. The resulting solution is stirred for 20 minutes at -78°C, during which time heavy precipitation occurs and uniform stirring becomes difficult. A solution of 1.33 g of p-toluenesulfonyl azide in 5 ml of tetrahydrofuran is added dropwise. The resulting mixture is allowed to stir at -78°C for 20 minutes, and 2 ml of trimethylsilyl chloride is added dropwise. The reaction mixture is warmed to ambient temperature and stirred for 1 hour. Then the mixture is cooled to 0°C and poured into 150 ml of 0°C ethyl acetate. Enough 0.5 M monobasic potassium phosphate buffer is added to make both the aqueous and organic layers clear. The two layers are separated and the organic layer is washed with three 150 ml portions of 0.5 M monobasic potassium phosphate solution, one 150 ml portion of sodium chloride solution, one 150 ml portion of saturated sodium chloride solution and dried over sodium sulfate. The solution is concentrated in vacuo to 2.83 g of oil, which upon trituration with hexane yields 1.67 g of the title compound as a solid.

C) <u>(±)-3-Azido-4,4-dimethyl-2-oxo-1-azetidine</u>

In a 50 ml three-necked flask, 1.52 g of (±)-3-azido-4,4-dimethyl-2-oxo-1-azetidine-tertbutyldiphenylsilane is dissolved in 25 ml of acetonitrile. To this stirred solution is added 0.25 ml of 48% hydrofluoric acid. This is stirred at ambient temperature, and 0.5 ml portions of 48% hydrofluoric acid are added every 60 minutes until, after 6.5 hours, a total of 3.25 ml of 48% hydrofluoric acid has been added. The reaction mixture is then cooled to 0°C, neutralized with saturated sodium bicarbonate, and extracted with 120 ml of ethyl acetate. The organic layer is then washed with 100 ml of water, 100 ml of saturated sodium chloride solution, and dried over sodium sulfate. The dry solution is concentrated *in vacuo* to yield 1.34 g of oil. This impure oil is chromatographed on 27 g of silica gel with hexane, followed by 33% ethyl acetate in hexane, to yield 0.358 g of the title compound as a solid.

D) <u>(±)-3-Azido-4,4-dimethyl-2-oxo-1-azetidinesulfonic acid, tetrabutylammonium salt</u>

To 0.100 g of (±)-3-azido-4,4-dimethyl-2-oxo-1-azetidine at 0°C is added under argon 2.8 ml of 0.5M dimethylformamide-sulfur trioxide complex. This mixture is allowed to warm to ambient temperature and stir for 45 minutes. The solution is then poured into 20 ml of 0.5 M pH 5.5 monobasic potassium phosphate buffer.

This is washed with three 20 ml portions of methylene chloride (discarded) and 0.237 g of tetrabutylammonium hydrogen sulfate is added to the aqueous solution. This was extracted with four 20 ml portions of methylene chloride and the combined organic extracts are washed with 20 ml of 8% sodium chloride solution. The methylene chloride solution is dried (sodium sulfate) and concentrated *in vacuo* to yield 0.31 g of oil, which appeared by nmr to be 50% dimethylformamide and 50% of the title compound.

E) (±)-3-Amino-4,4-dimethyl-2-oxo-1-azetidinesulfonic acid

A solution of 0.155 g of (±)-3-azido-4,4-dimethyl-2-oxo-1-azetidinesulfonic acid, tetrabutylammonium in 0.6 m l of methanol is hydrogenated over 10% palladium on charcoal for 20 minutes at 1 atmosphere. The catalyst is filtered off and rinsed with methylene chloride which is combined with the methanol solution. This clear solution is treated with 0.123 ml 97% formic acid. Upon addition of the acid the solution immediately becomes cloudy. After standing for 1 hour at 5°C, the solid is filtered off to yield 0.0664 g of the title compound, m.p. 200-202°C(dec.). NMR($D_2O$) 1.64(3H,S), 1.68(3H,S), 4.42(1H,S), IR(KBr) 1765$cm^{-1}$.

## Example 25

[3±(Z)]-3-[[(2-Amino-4-thiazolyl)(methoxyimino)-acetyl]amino]-4,4-dimethyl-2-oxo-1-azetidinesulfonic acid, potassium salt

A solution of N-hydroxybenzotriazole hydrate (50 mg) and (Z)-2-amino-α-(methoxyimino)-4-thiazoleacetic acid (0.323 mmole) in 0.5 ml of dimethylformamide is treated with 67 mg of dicyclohexylcarbodiimide under argon at ambient temperature. The resulting mixture is stirred for 1 hour at which time (±)-3-amino-4,4-dimethyl-2-oxo-1-azetidinesulfonic acid (57 mg; see example 153) is added as a solid followed by triethylamine dropwise (0.05 ml). The reaction is stirred at ambient temperature for 16 hours. The dimethylformamide is removed under high vacuum at 30°C, and the residue is slurried in 4 ml of acetone and filtered. The filter cake is washed with an additional 4 ml of acetone, and potassium perfluorobutanesulfonate (85 mg) is added to the filtrate, followed by ether. Trituration of the resulting gum with ether gives 40 mg of a tan solid which is chromatographed on a 70 ml HP-20AG column. Elution with water gives 20 mg of the title compound in fractions (5 ml) 16-40 after evaporation, trituration with 1:1 acetone-hexane and drying. m.p. 225°(dec).

Anal. Calc'd. for $C_{11}H_{14}N_5O_6S_2\cdot K$: C, 31.80; H, 3.40; N, 16.86; S, 15.43

Found: C, 29.47; H, 3.48; N, 14.98; S, 13.35

Example 26

(±)-4,4-Dimethyl-2-oxo-3-[(phenylacetyl)amino]-1-azetidinesulfonic acid, potassium salt

A solution of N-hydroxybenzotriazole hydrate (45 mg) and phenylacetic acid (40 mg) in 0.5 ml of dimethylformamide is treated with dicyclohexylcarbodiimide (61 mg) under argon at ambient temperature. The resulting mixture is stirred for 1 hour and (±)-3-amino-4,4-dimethyl-2-oxo-1-azetidinesulfonic acid, (52 mg; see example 153) is added as a solid, followed by triethylamine dropwise (0.04 ml). The reaction is stirred at ambient temperature for 24 hours. The dimethylformamide is removed under high vacuum at 30°C and the residue is slurried in acetone and filtered. Potassium perfluorobutanesulfonate is added to the filtrate, ether is added and the mixture is cooled. The resulting solid is washed with acetone, hexane, and dried yielding the title compound as a powder.

Anal. calc'd. for $C_{13}H_{15}N_2O_5SK$: C, 44.55; H, 4.32; N, 8.00; S, 9.15; K, 11.16

Found: C, 43.83; H, 4.16; N, 7.96; S, 8.76; K, 11.43

NMR($D_2O$) 1.33(S,3H), 1.58(S,3H), 3.68(S,3H), 4.70 (S,1H), 7.56ppm (broad S, 5H).

Example 27

(3S-trans)-3-[[(2-Amino-4-thiazolyl)oxoacetyl]-amino]-4-methyl-2-oxo-1-azetidinesulfonic acid, potassium salt

To a solution of diphenylphosphinyl chloride (1.85 g) in dry dimethylformamide (15 ml) cooled in an ice-methanol bath (-15° to -20°C) is added (2-amino-4-thiazolyl)glyoxylic acid, triethylamine

salt (2.14 g). After stirring for 0.5 hour a solution of (3S-trans)-3-amino-4-methyl-2-oxo-1-azetidinesulfonic acid (1.08 g; see example 139) and triethylamine (1.92 ml) in dry dimethylformamide (5 ml) is added to the cold mixed anhydride solution and the reaction mixture is stirred at 5°C for 24 hours. Solvent is removed *in vacuo*, the residual dark oil is dissolved in water, and chromatographed on Dowex 50 X 2-400 resin ($K^{\oplus}$ form, 200 ml). Upon elution with water (15 ml fractions) the crude product is collected in fractions 13-27 (3.37 g). Chromatography on HP-20 resin (200 ml), eluting with water (15 ml fractions), gives the desired product in fractions 18-26. Removal of water *in vacuo* gives the title compound as an amorphous powder.

*Anal*. *Calc'd* for $C_9H_9N_4O_6S_2K$ (372.42): C, 29.02; H, 2.44; N, 15.04; S, 17.22; K, 10.50

Found: C, 28.87; H, 2.62, N, 14.85; S, 15.09; K, 10.81

Example 28

(3S-trans)-3-Methoxy-4-methyl-2-oxo-3-[[(phenylmethoxy)carbonyl]amino]-1-azetidinesulfonic acid, potassium salt

A) (3S-trans)-4-Methyl-3-methoxy-2-oxo-4-[[(phenylmethoxy)carbonyl]amino]azetidine

A solution of 2.5 g (0.0106 mole) of (3R-trans)-4-methyl-2-oxo-3[[(phenylmethoxy)-carbonyl]amino]azetidine (prepared from d-threonine in 12.6% yield essentially as described for the racemic cis isomer in Example 98C) in 112 ml of 4% borax in methanol is cooled to 0°C and 3.5 ml of t-butyl hypochlorite is added. After 20 minutes the solution is poured into 1 liter of cold water and extracted with two 750 ml portions of cold ethyl acetate. The organic layer is washed with cold water (two 750 ml portions), saturated salt, dried and evaporated to give 3.05 g of crude N,N'-dichloroamide.

A solution of 426 mg of lithium methoxide in 20 ml of dry methanol is cooled to -78°C and diluted with 40 ml of dry tetrahydrofuran. Over 30 seconds a solution of the above chloroamide in 20 ml of tetrahydrofuran (-78°C) are added via syringe. After 20 minutes at -78°C, 2 ml each of acetic acid and trimethyl phosphite are added. After 40 minutes at room temperature the solution is poured into 500 ml of water and extracted with ethyl acetate (two 300 ml portions). The organic layer is washed with

water, dried, and evaporated to give an oil. Chromatography on a 200 ml silica gel column eluting with 3:1 chloroform-ethyl acetate gives a total of 1.25 g of the title compound.

B) (3S-*trans*)-3-Methoxy-4-methyl-2-oxo-3-[[(phenylmethoxy)carbonyl]amino-1-azetidinesulfonic acid, potassium salt

A solution of 800 mg (0.00303 mole) of (3S-*trans*)-4-methyl-3-methoxy-2-oxo-4-[[(phenylmethoxy)carbonyl]amino]azetidine in 2 ml of dimethylformamide is cooled to $0^{O}C$ and 4 ml of dimethylformamide-sulfur trioxide complex is added. After 1 hour at $0^{O}C$ and 4 hours at room temperature the solution is poured into 80 ml of 0.5 M monobasic potassium phosphate (adjusted to pH 5.5) and extracted with methylene chloride (two 50 ml portions, discard). The aqueous layer is treated with 1.04 g of tetrabutyl ammonium sulfate and extracted with dichloromethane to give 1.42 g of oil. This is dissolved in acetone and treated with 1.04 g of potassium perfluorobutanesulfonate in 10 ml of acetone. Dilution with 250 ml of ether and extensive trituration of the oily solid gives 584 mg of crude product. Chromatography on HP-20 AG (200 ml) gives 418 mg of purified product in fractions (100 ml) 13-16 (elution with 1 liter of water and then 9:1 water-acetone). Trituration of 114 mg of this material with ether gives 104 mg of an analytical sample.

Analysis calc'd for $C_{13}H_{14}N_2O_7SK \cdot H_2O$: C, 39.06; H, 4.04; N, 7.01; S, 8.03; K, 9.78

Found: C, 38.91; H, 3.62; N, 6.91; S, 8.06; K, 9.51

NMR($D_{2}0$) 1.33(3H, d, J=7), 3.46(3H,S), 4.22(2H, d of d, J=6), 5.18(2H,S), 7.43ppm(5H,S).

Example 29

(3S-trans)-3-Methoxy-4-methyl-2-oxo-3-[(phenylacetyl)-amino]-1-azetidinesulfonic acid, potassium salt

(3S-trans)-3-amino-3-Methoxy-4-methyl-2-oxo-1-azetidinesulfonic acid, tetrabutylammonium salt is prepared by the catalytic hydrogenation of (3S-trans)-3-methoxy-4-methyl-2-oxo-3-[[(phenylmethoxy)carbonyl]amino]-1-azetidinesulfonic acid, potassium salt (see example 158) after conversion to the tetrabutylammonium salt. Following the procedure of example 88, but utilizing (3S-trans)-3-amino-3-methoxy-4-methyl-2-oxo-1-azetidinesulfonic acid, tetrabutylammonium salt and phenylacetyl chloride yields the title compound.

Anal. calc'd for $C_{13}H_{15}N_{2}O_{6}SK$: C,42.61; H,4.31; N,7.65
Found: C,39.67; H,4.09; N,7.30

NMR ($D_{2}0$) 1.29(BH, d, j=7) 3.45(3H,S), 3.73(2H,S), 4.36 (2H, d of d, j=6), 7.38ppm (5H,S).

Example 30

[3S-[3α(Z),4β]]-3-[[(2-Amino-4-thiazolyl)[[2-(diphenylmethoxy)-2-oxoethoxy]imino]acetyl]-amino]-4-methyl-2-oxo-1-azetidinesulfonic acid, potassium salt

Following the procedure of example 9, but substituting (Z)-2-amino-α-[[2-(diphenylmethoxy)-2-oxoethoxy]imino]-4-thiazoleacetic acid for (Z)-2-amino-α-(methoxyimino)-4-thiazoleacetic acid and first treating the (3S-cis)-3-amino-4-methyl-2-oxo-1-azetidinesulfonic acid, with triethylamine, yields the title compound, melting point 155-160°C, dec.

Example 31

[3S-[3α(Z),4β]]-3-[[[(Carboxymethoxy)imino](2-amino-4-thiazolyl)acetyl]amino]-4-methyl-2-oxo-1-azetidinesulfonic acid, dipotassium salt

The deprotection of [3S-[3α(Z),4β]]-3-[[(2-amino-4-thiazolyl)[[2-(diphenylmethoxy)-2-oxoethoxy]-imino]acetyl]amino]-2-methyl-4-oxo-1-azetidine-sulfonic acid, potassium salt (see example 160) using trifluoroacetic acid and anisole yields the title compound, which decomposes at >250°C.

Example 32

[3S(R*)]-3-[[[[(4-Amino-2,3-dioxo-1-piperazinyl)-carbonyl]amino]phenylacetyl]amino]-4-methyl-2-oxo-1-azetidinesulfonic acid, potassium salt

[3S(R*)]-[4-[[[2-[(4-methyl-2-oxo-1-sulfo-3-azetidinyl)amino]-2-oxo-1-phenylethyl]amino]-carbonyl]-2,3-dioxo-1-piperazinyl]carbamic acid, phenylmethyl ester, potassium salt (see example 149) is hydrogenated using gaseous hydrogen and 10% palladium on charcoal as catalyst, yielding the title compound, melting point 165°C, dec.

Example 33

(trans)-3-Amino-4-ethyl-2-oxo-1-azetidinesulfonic acid

A) t-Boc-N-methoxy-β-threoethylserinamide

threo-D,L-β-Ethylserine (1.33 g) is dissolved in 10 ml of 2N potassium hydroxide and 5 ml of t-butanol. After the addition of 2.46 g of di-t-butylpyrocarbonate the two-phase mixture is stirred for 4 hours at ambient temperature. O-Methylhydroxylammonium chloride (1.25 g) is added and the pH is adjusted to 4 with 1N hydrochloric acid. 1-Ethyl-3-(3-dimethylamino-propyl)carbodiimide, hydrochloride (1.92 g) is added and the pH is again adjusted to 4. After stirring for 1 hour the reaction mixture is saturated with sodium chloride and extracted with four 50 ml portions of ethyl acetate. The ethyl acetate extracts are combined and dried over $MgSO_4$. Removal of the solvent *in vacuo* yields 1 g of the title compound.

B) t-Boc-O-methanesulfonyl-N-methoxy-β-threo-propionamide

t-Boc-N-methoxy-β-threoethylserinamide (10.5 g) is dissolved in 65 ml of pyridine. Methanesulfonyl chloride (4.65 ml) is added dropwise at 0°C. After stirring for 3 hours at ambient temperature, the reaction mixture is poured into 200 g of ice and 300 ml of 1N hydrochloric acid. The pH is adjusted to 4 with concentrated hydrochloric acid. After extraction with three 85 ml portions

of ethyl acetate the combined extracts are dried over $MgSO_4$ and concentrated *in vacuo*. The residue is treated with carbon tetrachloride and concentrated again. Stirring with ether followed by filtration yields 6.9 g of the title compound.

C) (*trans*)-3-*t*-Butoxycarbonylamino-4-ethyl-1-methoxy-2-azetidinone

Anhydrous potassium carbonate (4.15 g) and 125 ml of dry acetone are brought to reflux and 3.4 g of *t*-Boc-O-methanesulfonyl-N-methoxy-β-threo-propionamide in 25 ml of acetone is added. After 1 hour the reaction mixture is cooled and filtered, and the filtrate is concentrated *in vacuo*. The oily residue is stirred with hexane to yield 2.2 g of the title compound.

D) (*trans*)-3-*t*-Butoxycarbonylamino-4-ethyl-2-azetidinone

(*trans*)-3-*t*-Butoxycarbonylamino-4-ethyl-1-methoxy-2-azetidinone (3 g) is added to 170 ml of liquid ammonia at -78°C under nitrogen and 1.68 g of sodium is added in 5 portions with stirring over a 5 minute period. Stirring is continued for 30 minutes. Ammonium chloride is then added slowly until the blue color of the reaction mixture disappears. After removal of the ammonia under nitrogen the solid is extracted with two 100 ml portions of ethyl acetate. Removal of the solvent followed by drying *in vacuo* yields 2.7 g of the title compound.

E) (trans)-3-t-Butoxycarbonylamino-4-ethyl-2-oxo-1-azetidinesulfonic acid, tetrabutylammonium salt

To a 2 ml of absolute pyridine in 20 ml of dry dichloromethane is added trimethylsilylsulfonyl chloride (3.7 ml) in 5 ml of dry dichloromethane. The addition is accomplished at -30°C, under nitrogen, over a 10 minute period. After stirring at ambient temperature for 30 minutes, the flask is evacuated to yield a pyridine-sulfur trioxide complex. (trans)-3-t-Butoxycarbonylamino-4-ethyl-2-azetidinone (2.67 g) and 20 ml of dry pyridine are added to the flask which is then placed in an oil bath preheated to 90°C. After 15 minutes a clear solution is obtained and poured into 200 ml of a 1M solution of dibasic potassium phosphate. After the addition of 27 g of dibasic potassium phosphate and 100 ml of water a clear solution is obtained. The solution is extracted with two 60 ml portions of ethyl acetate. Tetrabutylammonium hydrogensulfate is added to the aqueous layer, and the aqueous solution is extracted with three 100 ml portions of dichloromethane and the combined organic layers are dried over $MgSO_4$. Concentration *in vacuo* yields 6.9 g of the title compound.

F) (trans)-3-Amino-4-ethyl-2-oxo-1-azetidinesulfonic acid

(trans)-3-t-Butoxycarbonylamino-4-ethyl-2-oxo-1-azetidinesulfonic acid, tetrabutylammonium salt (6.75 g) in 40 ml of 98% formic acid is stirred for 3 hours at ambient temperature. Dichloromethane (60 ml) is added and the mixture is refrigerated for about 16 hours. The resulting precipitate is separated by filtration and then dried in vacuo to yield 0.85 g of the title compound, melting point 185°C, dec.

Example 34

(trans,Z)-3-[[(2-Amino-4-thiazolyl)[(1-carboxy-1-methylethoxy)imino]acetyl]amino]-4-ethyl-2-oxo-1-azetidinesulfonic acid, dipotassium salt

A) (trans,Z)-3-[[(2-Amino-4-thiazolyl)[(1-diphenylmethoxycarbonyl-1-methylethoxy)imino]-acetyl]amino]-4-ethyl-2-oxo-1-azetidinesulfonic acid, dipotassium salt

(trans)-3-Amino-4-ethyl-2-oxo-1-azetidinesulfonic acid (0.55 g) and 335 mg of triethylamine are dissolved in 50 ml of dry dimethylformamide. (Z)-2-Amino-α-[(1-diphenylmethoxycarbonyl-1-methylethoxy)imino]-4-thiazoleacetic acid (1.14 g) is added with stirring at 0°C followed by 450 mg of hydroxybenzotriazole and then 0.69 g of dicyclohexylcarbodiimide. After stirring for about 16 hours at 0°C the flask is evacuated. Dry acetone (25 ml) is added to the solid with

stirring. The mixture is filtered and 0.94 g of potassium perfluorobutanesulfonate is added to the filtrate followed by 100 ml of ether. After standing for 1 hour at 0°C the solid is filtered, washed with ether and dried *in vacuo* yielding 1.58 g of the title compound.

B) (trans,Z)-3-[[(2-Amino-4-thiazolyl)[(1-carboxy-1-methylethoxy)imino]acetyl]amino]-4-ethyl-2-oxo-1-azetidinesulfonic acid, dipotassium salt

To a suspension of 1.31 g of (trans),Z)-3-[[(2-amino-4-thiazolyl)](1-diphenylmethoxycarbonyl-1-methylethoxy)imino]acetyl]amino]-4-ethyl-2-oxo-1-azetidinesulfonic acid, dipotassium salt in 10 ml of anisole is added 5 ml of trifluoroacetic acid over a 10 minute period at -15°C. After stirring for 2 hours at -10°C a clear solution is obtained. At -30°C 80 ml of dry ether is added and the resulting precipitate is filtered and then treated with 5 ml of water. The pH is adjusted to 5.5 with 1N potassium hydroxide at 0°C, and the mixture is filtered to remove unconverted starting material. The filtrate is chromatographed on HP-20 with water as eluent. Lyophilization yields 185 mg of the title compound, melting point 160°C, *dec*.

Example 35

(3S-trans)-α-[[(4-Methyl-2-oxo-1-sulfo-3-azetidinyl)-amino]carbonyl]benzeneacetic acid, dipotassium salt

(3S-trans)-3-Amino-4-methyl-2-oxo-1-azetidinesulfonic acid (see example 139) is coupled with α-(carboxyl)benzeneacetyl chloride and treated with triethylamine and potassium perfluorobutanesulfonate to yield the title compound, melting point 147°C, dec.

Example 36

[3S-trans]-3-Amino-4-cyclohexyl-2-oxo-1-azetidinesulfonic acid

A) α-(t-Butoxycarbonylamino)-β-cyclohexyl-β-hydroxy-threo-propionic acid

β-Cyclohexyl-α-amino-β-hydroxy-threo-propionic acid (15 g) is suspended in 150 ml of acetonitrile and 70 ml of water. Triethylamine (17.8 g) is added and the mixture is heated with stirring to 60°C. At this temperature a clear solution is obtained and 21.0 g of di-t-butylpyrocarbonate is added and stirring at 60°C is continued for 1.5 hours. The solvent is removed in vacuo and 50 ml of water is added. The aqueous layer is extracted with ethyl acetate at a pH of 2, which is adjusted by addition of 3N HCl. The organic layer is separated, dried over $Na_2SO_4$ and evaporated to dryness. The remaining crystalline material is filtered with petrol ether, yielding 20.4 g of the title compound, melting point 113-115°C.

B) α-(t-Butoxycarbonylamino)-β-cyclohexyl-β-hydroxy-N-methoxy-threo-propionamide

α-(t-Butoxycarbonylamino)-β-cyclohexyl-β-hydroxy-threo-propionic acid (20.2 g) and 7.6 g of O-methylhydroxylamine hydrochloride are suspended in 350 ml of water and 175 ml of t-butanol. The pH of the mixture is adjusted with potassium carbonate to 4.1-Ethyl-3-(3-dimethylaminopropyl)carbodiimide (16.4 g) is added and the pH is maintained at 4 with stirring for 1.5 hours. t-Butanol is removed in vacuo and the remaining aqueous solution is saturated with sodium chloride and extracted twice with 100 ml portions of ethyl acetate. The organic layers are combined, dried with $Na_2SO_4$ and evaporated to dryness. The remaining crystals are filtered off with petrol ether yielding 18.6 g of the title compound, melting point 125-127°C.

C) α-(t-Butoxycarbonylamino)-β-cyclohexyl-β-(methanesulfonyloxy)-N-methoxy-threo-propionamide

α-(t-Butoxycarbonylamino)-β-cyclohexyl-β-hydroxy-N-methoxy-threo-propionamide (18.3 g) is dissolved with stirring in 100 ml of dry pyridine. The solution is cooled with stirring to 0°C and 9.3 g of methanesulfonyl chloride is dropped in. After one hour at 0°C an additional 3.3 g of methanesulfonyl chloride is added and stirring is continued for one more hour. The solution is poured into 300 ml of ice water,

200 ml of ethyl acetate is added and the pH is adjusted to 3 with dilute sulfuric acid. The organic layer is separated, dried with $Na_2SO_4$ and the solvent is removed *in vacuo*. The remaining solid is collected with petrol ether yielding 19.0 g of the title compound, melting point 150-152°C.

D) [3S-*trans*]-3-(*t*-Butoxycarbonylamino)-4-cyclohexyl-1-methoxy-2-azetidinone

α-(*t*-Butoxycarbonylamino)-β-cyclohexyl-β-(methanesulfonyloxy)-N-methoxy-threo-propionamide (18.7 g) is dissolved in 500 ml of dry acetone. Potassium carbonate (9.8 g) is added and the suspension is heated to reflux temperature with stirring for 5 hours. The insoluble inorganic material is filtered off and the solvent removed *in vacuo* and the remaining oil is dissolved in 30 ml of ethyl acetate. Upon the addition of petrol ether, the title compound precipitates and is filtered off (12.9 g), melting point 110-112°C.

E) [3S-*trans*]-3-(*t*-Butoxycarbonylamino)-4-cyclohexyl-2-azetidinone

[3S-*trans*]-3-(*t*-Butoxycarbonylamino)-4-cyclohexyl-1-methoxy-2-azetidinone (1 g) is added to 50 ml of liquid ammonia with stirring. Sodium (0.154 g) is added in 5 to 6 portions within 5 minutes. After this time an additional

amount of 0.025 g sodium is added and stirring is continued for 5 minutes. Ammonium chloride (0.89 g) is added and the ammonia is removed. The residue is extracted with warm ethyl acetate. The organic extract is evaporated to dryness and the remaining crystals of the title compound are filtered with petrol ether, yielding 0.5 g, melting point 130-132°C.

F) [3S-trans]-3-(t-Butoxycarbonylamino)-4-cyclohexyl-2-oxo-1-azetidinesulfonic acid, pyridine salt

[3S-trans]-3-(t-Butoxycarbonylamino)-4-cyclohexyl-2-azetidinone (5.3) is dissolved in 20 ml of methylene chloride and 80 ml of dimethylformamide. After the addition of 60 mmole of pyridine-sulfur trioxide complex the solution is stirred for 6 hours at room temperature. Removal of the solvent *in vacuo* yield 11.3 g of the title compound as an oil.

G) [3S-trans]-3-(t-Butoxycarbonylamino)-4-cyclohexyl-2-oxo-1-azetidinesulfonic acid, tetrabutylammonium salt

[3S-trans]-3-(t-Butoxycarbonylamino)-4-cyclohexyl-2-oxo-1-azetidinesulfonic acid, pyridine salt (11.3 g) is dissolved in 250 ml of water. Tetrabutylammonium hydrogensulfate (9.0 g) is added with stirring and the pH is adjusted to 6.5 with 1N potassium hydroxide. The aqueous solution is extracted twice with

200 ml portions of methylene chloride. The organic portions are dried with $Na_2SO_4$, filtered and the solvent is distilled off, yielding 8 g of the title compound, melting point 135-138°C.

H) [3S-trans]-3-Amino-4-cyclohexyl-2-oxo-1-azetidinesulfonic acid

[3S-trans]-3-(t-Butoxycarbonylamino)-4-cyclohexyl-2-oxo-1-azetidinesulfonic acid, tetrabutylammonium salt (3.8 g) is stirred in 20 ml of formic acid for 3 hours, followed by the addition of 20 ml of methylene chloride. The precipitated title compound (1.0 g) is filtered off, melting point 217-219°C.

Example 37

[3S-[3α(Z),4β]]-3-[[(2-Amino-4-thiazolyl)-(methoxyimino)acetyl]amino]-4-cyclohexyl-2-oxo-1-azetidinesulfonic acid, potassium salt

[3S-trans]-3-Amino-4-cyclohexyl-2-oxo-1-azetidinesulfonic acid (0.25 g) is dissolved in 30 ml of dry dimethylformamide and 0.12 g of triethylamine with stirring. When a clear solution is obtained, (Z)-2-amino-α-(methoxyimino)-4-thiazoleacetic acid (0.2 g), 0.16 g of hydroxybenzotriazole and 0.42 g of dicyclohexylcarbodiimide are added. Stirring is continued for 48 hours at ambient temperature. The precipitated urea is filtered off and the solvent is removed *in vacuo*. The residue is

dissolved in 10 ml of acetone and 0.41 g of potassium perfluorobutanesulfonate is added. After the addition of 50 ml of ether, the title compound precipitates and is filtered. Column chromatography using HP-20 and water/acetone (9:1) as eluent, yields 0.36 g of product, melting point 200-205°C (after lyophilization).

## Example 38

[3S-[3α(Z),4β]]-3-[[(2-Amino-4-thiazolyl)[(1-carboxy-1-methylethoxy)imino]acetyl]amino]-4-cyclohexyl-2-oxo-1-azetidinesulfonic acid, dipotassium salt

A) [3S-[3α(Z),4β]]-3-[[(2-Amino-4-thiazolyl)[(1-diphenylmethoxycarbonyl-1-methylethoxy)imino]-acetyl]amino]-4-cyclohexyl-2-oxo-1-azetidinesulfonic acid, potassium salt

[3S-trans]-3-Amino-4-cyclohexyl-2-oxo-1-azetidinesulfonic acid (0.2 g; see example 175) is dissolved in 30 ml of dimethylformamide and 0.09 g of triethylamine with stirring. Hydroxybenzotriazole (0.12 g), 0.30 g of (Z)-2-amino-α-[(1-diphenylmethoxycarbonyl-1-methylethoxy)imino]-4-thiazoleacetic acid and 0.33 g of dicyclohexylcarbodiimide are added and stirring at ambient temperature is continued for 12 hours. The precipitated urea is filtered off and the mother liquor is evaporated to dryness. The remaining oil is dissolved in 5 ml acetone, treated with 0.3 g of potassium perfluorobutanesulfonate and

poured into 100 ml of ether with stirring. [3S-[3α(Z),4β]]-3-[[(2-Amino-4-thiazolyl)-[(1-diphenylmethoxycarbonyl-1-methylethoxy)-imino]acetyl]amino]-4-cyclohexyl-2-oxo-1-azetidine-sulfonic acid, potassium salt (0.61 g) precipitates and is filtered off.

B) [3S-[3α(Z),4β]]-3-[[(2-Amino-4-thiazolyl)[(1-carboxy-1-methylethoxy)imino]acetyl]amino]-4-cyclohexyl-2-oxo-1-azetidinesulfonic acid, dipotassium salt

[3S-[3α(Z),4β]]-3-[[(2-Amino-4-thiazolyl)-[(1-diphenylmethoxycarbonyl-1-methylethoxy)imino]-acetyl]amino]-4-cyclohexyl-2-oxo-1-azetidinesulfonic acid, potassium salt (0.61 g) is suspended in 6 ml of anisole, cooled to -15°C and 5 ml of trifluoroacetic acid is dropped in with stirring. The temperature is maintained for one hour and then lowered to -30°C. About 100 ml of dry ether is added at such a rate, that the temperature does not exceed -10°C. The precipitated compound is filtered off and chromatographed using HP-20 resin and water/acetone (9:1) as eluent, yielding 0.3 g of the title compound, melting point 11-120°C, dec. (after lyophilization).

Example 39

[3S-[3α,4β]-4-Cyclohexyl-3-[[[[3-[(2-furanyl-methylene)amino]-2-oxo-1-imidazolidinyl]carbonyl]-amino]phenylacetyl]amino]-2-oxo-1-azetidinesulfonic acid, potassium salt

[3S-*trans*]-3-Amino-4-cyclohexyl-2-oxo-1-azetidinesulfonic acid (0.1 g; see example 175) is dissolved in a mixture of 30 ml of dry dimethylformamide and 0.05 g of triethylamine with stirring. [[[(2-Furanylmethylene)amino]-2-oxo-1-imidazolidinyl]carbonyl]amino]phenylacetic acid (0.14 g), 0.06 g hydroxybenzotriazole and 0.17 g of dicyclohexylcarbodiimide are added and the solution is stirred for 5 days at room temperature. The solvent is removed *in vacuo*, the residue is dissolved in 10 ml of acetone and the precipitated urea is filtered off. The mother liquor is agitated with 0.15 g of potassium perfluorobutanesulfonate and diluted with 50 ml of ether. The precipitate is filtered, and chromatographed with HP-20 resin using water/acetone (9:1) as eluent yielding 0.14 g of product, melting point 195-200°C, *dec*. (after lyophiliaztion).

Example 40

[3S-[3α(R*),4β]]-4-Cyclohexyl-3-[[3-(4-ethyl-2,3-dioxo-1-piperazinyl)-1.3-dioxo-2-phenylpropyl]-amino]-2-oxo-1-azetidinesulfonic acid, potassium salt

[3S-trans]-3-Amino-4-cyclohexyl-2-oxo-1-azetidinesulfonic acid (0.1 g; see example 175) is dissolved in 30 ml of dimethylformamide and 0.5 g of triethylamine with stirring. (R)-α-[[(4-ethyl-2,3-dioxo-1-piperazinyl)carbonyl]-amino]benzeneacetic acid, 0.06 g of hydroxybenzotriazole and 0.17 g of dicyclohexylcarbodiimide are added, and the mixture is stirred for about 16 hours at room temperature. The solvent is distilled off *in vacuo* and the remaining oil is dissolved in 10 ml of acetone. The precipitated urea is filtered off and the mother liquor is agitated with 0.15 g of potassium perfluorobutanesulfonate and diluted with 50 ml of ether. The precipitate is filtered off and chromatographed with HP-20 resin, using water/acetone (9:1) as eluent, yielding 0.15 g of the title compound, melting point 175-180°C (after lyophilization).

Example 41

(trans,Z)-3-[[(2-Amino-4-thiazolyl)(methoxyimino)-acetyl]amino]-4-ethyl-2-oxo-1-azetidinesulfonic acid, potassium salt

Following the procedure of example 170, part A, but substituting (Z)-2-amino-α-(methoxyimino)-4-thiazoleacetic acid for (Z)-2-amino-α-[(1-diphenylmethoxycarbonyl-1-methylethoxy)imino]-4-thiazoleacetic acid, yields the title compound, melting point 190°C, *dec*.

Example 42

(±)-(trans)-3-Amino-2-oxo-4-phenyl-1-azetidinesulfonic acid

A) (±)-(trans)-2-oxo-4-phenyl-1-azetidine-tert-butyldiphenylsilane

A solution of tert-butylchlorodiphenylsilane (20.56 g) in dimethylformamide (45 ml) is cooled to 0°C under argon and treated with triethylamine (10.4 ml) and then (±)-2-oxo-4-phenyl-1-azetidine. After several hours at 0°C, the resulting mixture is treated with additional triethylamine (1 ml) and tert-butylchlorodiphenylsilane (2.11 g), and allowed to stir for 65 hours at 5°C. The reaction mixture is poured into ice water (300 ml) and extracted with 3:1 ether-ethyl acetate (three 125 ml portions). The organic extracts are washed with pH 4.5 phosphate buffer (three 50 ml portions), saturated sodium bicarbonate solution (50 ml), water (two 50 ml portions) saturated sodium chloride solution and dried ($Na_2SO_4$). Filtration, and concentration *in vacuo* yields a solid which is washed with hexane to give after drying (high vacuum) 15 g of the title compound as a solid.

B) <u>(±)-(trans)-3-azido-2-oxo-4-phenyl-1-azetidine-tert-butyldiphenylsilane</u>

A 50 ml flask equipped with stirring bar, gas inlet, and septum is flame dried under argon and charged with n-butyl lithium (0.65 ml of a 1.6 M solution in hexane) which is cooled to -40°C and dissolved in tetrahydrofuran (2 ml). Diisopropylamine (0.16 ml) is added dropwise, the resulting mixture is stirred for 30 minutes and cooled to -78°C. A solution of (±)-(trans)-2-oxo-4-phenyl-1-azetidine-tert-butyldiphenylsilane (400 mg) in tetrahydrofuran (1.5 ml) is added dropwise over about 5 minutes. After stirring an additional 20 minutes the solution is treated with p-toluenesulfonyl azide (204 mg) in tetrahydrofuran (0.5 ml). The resulting mixture is stirred 10 minutes at -78°C and treated dropwise with chlorotrimethylsilane (0.4 ml). After an additional 10 minutes of stirring, the cooling bath is removed and the reaction mixture is stirred at ambient temperature for 2.5 hours. Then, while cooling at 0°C, ethyl acetate (20 ml) is added followed by pH 4.5 phosphate buffer (8 ml). The organic layer is washed with additional buffer (two 8 ml portions), 5% sodium bicarbonate solution (three 10 ml portions), 50% sodium chloride solution (10 ml), saturated sodium chloride solution (10 ml) and dried ($Na_2SO_4$). Filtration and concentration *in vacuo* yields 500 mg of oil which is flash chromatographed with 5% ethyl acetate-hexane, yielding the title compound (253 mg).

C) (±)-(trans)-3-azido-2-oxo-4-phenyl-1-azetidine

A solution of 17 g of crude (±)-(trans)-3-azido-2-oxo-4-phenyl-1-azetidine-tert butyldiphenylsilane is dissolved in methanol (240 ml) and treated dropwise at 0°C with concentrated HCl (35 ml). The cooling bath is removed, the reaction stirred at ambient temperature for 1 hour, and recooled to 0°C whereupon saturated sodium bicarbonate solution is added to neutrality. The resulting mixture is extracted with ethyl acetate (one 300 ml portion and four 100 ml portions) and the organic extracts are washed with 1:1 5% sodium bicarbonate 50% sodium chloride solution, saturated sodium chloride solution, and dried ($Na_2SO_4$). Filtration and concentration *in vacuo* yields 15 g of a heavy oil which is chromatographed on 100 g of silica gel with 20% ethyl acetate-hexane, yielding 460 mg of the title compound.

D) (±)-(trans)-3-azido-4-phenyl-1-azetidinesulfonic acid, tetrabutylammonium salt

A solution of (±)-(trans)-3-azido-2-oxo-4-phenyl-1-azetidine (300 mg) in dimethylformamide (3 ml) is cooled to 0°C under argon and treated dropwise with a complex of dimethylformamide and sulfur trioxide (4.78 ml of a 0.5 M solution in dimethylformamide). The cooling bath is removed, the reaction mixture is stirred at ambient temperature for 2 hours

and poured into 80 ml of 0.5 M monobasic potassium phosphate (pH 5.5). The solution is extracted with dichloromethane (discarded) and 541 mg of tetrabutylammonium bisulfate is added. The resulting mixture is extracted with dichloromethane and the organic extracts are washed with 10% sodium chloride solution and dried ($Na_2SO_4$). Filtration and concentration *in vacuo* affords 800 mg of oil; approximately 40% the desired product, the remainder dimethylformamide. This mixture is used without purification in the next step.

E) (±)-(*trans*)-3-amino-4-phenyl-1-azetidinesulfonic acid

A solution of (±)-(*trans*)-3-amino-4-phenyl-1-azetidinesulfonic acid, tetrabutylammonium salt in 4 ml of methanol is hydrogenated over 30 mg of platinum oxide at 1 atmosphere and room temperature. After 15 minutes, the system is evacuated and fresh hydrogen is introduced. After an additional 45 minutes the reaction is complete, and the system is flashed with nitrogen. After several days at room temperature in dichloromethane-methanol (4:1, 200 ml) catalyst aggregation is complete and filtration is accomplished. The filtrate is concentrated *in vacuo* to 18 ml and 0.2 ml of 97% formic acid is added. After cooling at 5°C for several hours the resulting solid is filtered and washed with dichloromethane to afford, after drying, 150 mg of the title compound as a solid.

Analysis calc'd for $C_9H_{19}N_2O_4S$: C, 44.62; H, 4.17; N, 11.57; S, 13.23

Found: C, 43.36; H, 4.31; N, 11.09; S, 13.02

## Example 43

### (±)-(trans)-2-Oxo-4-phenyl-3-[(phenylacetyl)amino]-1-azetidinesulfonic acid, potassium salt

A) (±)-trans-2-oxo-4-phenyl-3-[(phenylacetyl)amino]-1-azetidinesulfonic acid tetrabutylammonium salt

A solution of N-hydroxybenzotriazole monohydrate (52 mg) and phenylacetic acid (46 mg) in dimethylformamide (0.3 ml) is treated with solid dicyclohexylcarbodiimide (70 mg) under argon at 0°C. The cooling bath is removed and the resulting mixture stirred at ambient temperature for 1 hour. After dilution with additional dimethylformamide (0.3 ml), (±)-(trans)-3-amino-2-oxo-4-phenyl-1-azetidinesulfonic acid is added as a solid (75 mg, see example 181) followed by triethylamine (0.05 ml) dropwise. The reaction is stirred at ambient temperature for 23 hours, filtered and the filter cake washed with dimethylformamide. The filtrate was added to 20 ml 0.5 monobasic potassium phosphate (pH 4.5), the mixture washed with three 8 ml portions of ethyl acetate (discard), and 105 mg of tetrabutylammonium bisulfate (0.31 mmole) is added. The resulting mixture is extracted with dichloromethane (three 15 ml portions). The extracts are washed with 10% sodium chloride solution (two 15 ml portions), saturated sodium chloride solution (10 ml) and dried ($Na_2SO_4$). Filtration and concentration *in vacuo* yields (after heating at 32°C under high vacuum) 165 mg of oil; approximately 40% is the desired compound, and 60% dimethylformamide.

B) (±)-trans-2-oxo-4-phenyl-3-[(phenylacetyl)-amino]-1-azetidinesulfonic acid, potassium salt

A solution of (±)-*trans*-2-oxo-4-phenyl-3-[(phenylacetyl)amino]-1-azetidinesulfonic acid tetrabutylammonium salt in 1.5 ml acetone is treated with 41 mg (0.121 mmole) of potassium perfluorobutanesulfonate. Dilution with 12 ml of ether affords a glass, which upon trituration with ether affords 43 mg of solid which contains about 20% of an impurity containing a tetrabutylammonium moiety. The solid is dissolved in 50% aqueous acetone and passed through a Dowex 50W-X2 $K^{\oplus}$ ion-exchange resin (1 ml). Removal of solvent yields a solid which is washed with acetone, hexane and dried (60°C, high vacuum). The yield of the title compound is 15 mg.

Analysis calc'd for $C_{17}H_{15}N_2O_5S \cdot K$: C, 51.23; H, 3.80; N, 7.03; S, 8.05; K, 9.81

Found: C, 50.44; H, 4.20; N, 7.01; S, 7.59; K, 9.40

## Example 44

(±)-(*trans*,Z)-3-[[2-Amino-4-thiazolyl)(methoxyimino)-acetyl]amino]-2-oxo-4-phenyl-1-azetidinesulfonic acid, potassium salt

A solution of N-hydroxybenzotriazole hydrate (52 mg) and (Z)-2-amino-α-(methoxyimino)-4-thiazoleacetic acid (69 mg) in dimethylformamide (0.3 ml) is treated with solid dicyclohexylcarbodiimide (70 mg) under argon, at ambient

temperature. The resulting mixture is stirred for 1 hour, (±)-(trans)-3-amino-2-oxo-4-phenyl-1-azetidinesulfonic acid (75 mg; see example 181) is added as a solid, followed by triethylamine dropwise (0.05 ml). The reaction is stirred at ambient temperature for 23 hours. The dimethylformamide is removed under high vacuum at 30°C, and the residue triturated with 2 ml of acetone and filtered. The filter cake is washed with additional acetone (two 3 ml portions) and potassium perfluorobutanesulfonate (86 mg) is added to the filtrate. Dilution with 10 ml of ether produces a gummy solid which is triturated, washed with acetone and hexane to yield, after drying, 82 mg of the title compound as a solid.

Analysis for $C_{15}H_{14}N_5O_6S_2 \cdot K$

Calc'd: C, 40.26; H, 3.16; N, 15.65; S, 14.33; K, 8.74

Found: C, 38.60; H, 3.19; N, 15.07; S, 13.87; K, 7.5

Example 45

(cis)-2-Oxo-4-phenyl-3-[(phenylacetyl)amino]-1-azetidinesulfonic acid, potassium salt

A) N-Benzylidene-2,4-dimethoxybenzylamine

12.0 g of 2,4-dimethoxybenzylamine hydrochloride is added to 100 ml of 1N sodium hydroxide solution and the mixture is extracted with 125 ml of ethyl acetate. The organic layer is dried over anhydrous sodium sulfate and stripped of solvent to give

10.2 g of 2,4-dimethoxybenzylamine as an oil. This amine is dissolved in 150 ml of benzene; 6.47 g of benzaldehyde and 0.6 g of p-toluenesulfonic acid monohydrate are added. The mixture is heated under reflux removing water with a Dean-Stark separator and in two hours the calculated amount of water (1.1 ml) separates out. The mixture is cooled to room temperature. Upon further cooling the benzene solution deposits some precipitate. Benzene is removed under reduced pressure and 60 ml of petroleum ether is added to the residue. An oily layer separates out with more precipitate. Benzene (10 ml) is added to make the layers homogeneous and the remaining precipitate is filtered. The filtrate is stripped of solvent to give 14.2 g of the title compound as an oil.

B) (±)-(cis)-4-Phenyl-1-(2,4-dimethoxybenzyl)-2-oxo-3-azidoazetidine

α-Azidoacetic acid (1.62 g) is dissolved in 25 ml of methylene chloride under nitrogen. To this solution are added 3.24 g of triethylamine and 1.02 g (4.0 mmole) of the imine N-benzylidene-2,4-dimethoxybenzylamine dissolved in 10 ml of methylene chloride. The mixture is cooled in an ice-bath and 3.36 g of trifluoroacetic anhydride is added slowly; the solution becomes dark colored. After stirring for 1 hour in an ice-bath, the mixture is warmed to room temperature and stirred an additional 15 minutes.

The solution is then washed with water (60 ml), 5% $NaHCO_3$ solution (two 50 ml portions), and 1N HCl solution (60 ml). The organic layer is dried over anhydrous sodium sulfate and stripped of solvent to give 1.72 g of crude product as dark gum. The gum is treated with charcoal several times and the resulting brown mixture is chromatographed on 40 g silica gel eluting with 1:1 petroleum ether:ethyl acetate. The combined fractions yield crystal upon quick-freezing in a dry ice-acetone bath. Using this as a seed the product is recrystallized from petroleum ether-ethyl acetate to give 817 mg of the title compound as needles which melt upon warming to room temperature.

C) (±)-(cis)-4-Phenyl-2-oxo-3-azidoazetidine

(±)-(cis)-4-Phenyl-1-(2,4-dimethoxybenzyl)-2-oxo-3-azidoazetidine (737 mg) is dissolved in 25 ml of acetonitrile and heated to 80°-83°C under nitrogen. To the resulting solution are added over a 1 hour period 943 mg of potassium persulfate and 570 mg of potassium monohydrogen phosphate, both dissolved in 25 ml of water. After the addition, the mixture is further heated at 80°-83°C for 7 hours. The mixutre is cooled and the pH is adjusted to 6-7 by adding solid potassium monohydrogen phosphate. Most of the acetonitrile is removed under reduced pressure and the resulting mixture is extracted with

60 ml of chloroform. The chloroform layer is washed with water (60 ml), dried over anhydrous sodium sulfate and stripped of solvent to give a crude product as an oil. The crude product is chromatogrpahed on 40 g of silica gel eluting with 1:1 petroleum ether-ethyl acetate. The combined fractions yield crystals and the product is recrystallized from petroleum ether-ethyl acetate to give 267 mg of the title compound.

D) (±)-(cis)-4-Phenyl-2-oxo-3-azido-1-azetidinesulfonic acid, tetrabutyl ammonium salt

(±)-(cis)-4-Phenyl-2-oxo-3-azidoazetidine (162 mg) is cooled to 0°C under nitrogen and 3.5 ml of ca0.5M dimethylformamide-sulfur trioxide complex solution in dimethylformamide is added dropwise via syringe. The resulting clear solution is stirred at 0°C for 15 minutes. The mixture is then poured into 50 ml of 0.5M monobasic potassium phosphate solution and washed with methylene chloride (three 50 ml portions). tetra-n-Butylammonium bisulfate (292 mg) is added to the aqueous solution and the mixture is extracted with methylene chloride (six 50 ml portions). The combined methylene chloride layers are dried over anhydrous sodium sulfate and stripped of solvent to give 272 mg of the title compound as a gum.

E) (±)-(cis)-2-Oxo-4-phenyl-3-[(phenylacetyl)amino]-1-azetidinesulfonic acid, potassium salt

(±)-(cis)-4-Phenyl-2-oxo-3-azido-1-azetidinesulfonic acid, tetrabutyl ammonium salt (293 mg) is dissolved in 4 ml of ethanol and hydrogenated with 80 mg of platinum oxide catalyst at one atmosphere. After 1 hour stirring the catalyst is filtered through a Millipore filter with Celite; some catalyst particles pass through the filter to give a black filtrate. Ethanol is removed under reduced pressure and the residue is dissolved in 4 ml of dimethylformamide. N-Hydroxybenzotriazole monohydrate (81 mg), 78 mg of phenylacetic acid, and 117 mg of dicyclohexylcarbodiimide are added and the mixture is stirred for about 16 hours under nitrogen. The slurry is evaporated *in vacuo* and triturated with 10 ml of acetone. The resulting slurry is filtered through a Millipore filter with a Celite top and the brown filtrate is treated with 193 mg of potassium perfluorobutane sulfonate. Upon adding 20 ml of ether a gum separates out. Liquid is removed and the gum is washed with ether. The gum is dissolved in 10 ml of methanol. Upon adding ether, a small amount of precipitate is formed. The mixture is filtered and the colored filtrate is treated with more ether. The precipitate formed is filtered and recrystallized twice from ether-methanol to give 26 mg of the title compound.

Analysis calc'd for $C_{17}H_{15}O_5N_2SK \cdot 2H_2O$:

C, 46.99; H, 4.41; N, 6.45

Found: C, 47.24; H, 4.19; N, 6.34

## Example 46

**(cis,Z)-3-[[(2-Amino-4-thiazolyl)(methoxyimino)-acetyl]amino]-2-oxo-4-phenyl-1-azetidinesulfonic acid, potassium salt**

(cis)-2-Oxo-4-phenyl-3-[(phenylacetyl)amino]-1-azetidinesulfonic acid, potassium salt (560 mg; see example 184, part D] is dissolved in 5 ml of ethanol and hydrogenated with 110 mg of platinum oxide catalyst at one atmosphere. After one hour stirring the catalyst is filtered through a Millipore filter with Celite; catalyst particles pass through the filter to give a black filtrate. Ethanol is removed under reduced pressure and the residue is dissolved in 4 ml of dimethylformamide. N-Hydroxybenzotriazole monohydrate (168 mg), 221 mg of the (Z)-2-amino-α-(methoxyimino)-4-thiazoleacetic acid and 227 mg of dicyclohexylcarbodiimide are added and the mixture is stirred for about 16 hours under nitrogen. The slurry is evaporated *in vacuo* and triturated with 15 ml of acetone. The resulting slurry is filtered through a Millipore with Celite and the filtrate is treated with 372 mg of potassium perfluorobutane sulfonate. Upon adding 15 ml of ether a gum separates out. Liquid is removed and the gum is washed with ether. The gum is dissolved in 5 ml of water and applied on 150 ml of HP-20 resin eluting with water. Fractions (30 ml each) 16-34 are combined and lyophilized to give 201 mg of the title compound as a solid.

Analysis calc'd for $C_{15}H_{14}O_6N_5S_2K \cdot 1\,1/2\ H_2O$:
C, 36.73; H, 3.49; N, 14.28; S, 13.07; K, 7.97
Found: C, 36.65; H, 3.00; N, 13.99; S, 13.48; K, 8.30

Example 47

(cis)-3-Amino-2-oxo-4-(2-phenylethenyl)-1-azetidinesulfonic acid

A) N-(3-phenyl-2-propenylidene)-4-methoxyaniline

p-Anisidine (12.32 g) is dissolved in 160 ml of methylene chloride and 20 g of anhydrous magnesium sulfate is added. The mixture is cooled in an ice bath and 13.22 g of trans-cinnamaldehyde is added. The mixture is stirred under nitrogen for 2 hours and then filtered. The filtrate is evaporated to give a solid. The crude product is recrystallized from methylene chloride-petroleum ether to give 20.96 y of the title compound as a solid.

B) (±)-(cis)-3-Azido-1-(4-Methoxyphenyl)-2-oxo-4-(2-phenylethenyl)azetidine

2-Azidoacetic acid (24.26 g) is dissolved in 100 ml of methylene chloride and cooled in an ice bath. To this solution is added 48.57 g of triethylamine and 14.24 g of N-(3-phenyl-2-propenylidene)-4-methoxyaniline dissolved in 250 ml of methylene chloride. To the resulting solution is added 50.41 g of trifluoroacetic anhydride dropwise over a one hour period. After stirring for one hour in an ice bath, the mixture is warmed to room temperature and stirred for about 16 hours. The mixture is then diluted with 250 ml of methylene chloride and washed

with water (750 ml), 5% sodium bicarbonate solution (two 750 ml portions), and 1N HCl solution (750 ml). The organic layer is dried over anhydrous sodium sulfate and stripped of solvent to give a solid. The crude product is recrystallized from ethyl acetate to give 11.39 g of the title compound as a solid.

C) (±)-(cis)-3-Azido-2-oxo-4-(2-phenylethenyl) azetidine

To a solution of 10.22 g of ceric ammonium nitrate in 13 ml of water at 0°C is added 1.99 g of (±)-(cis)-3-azido-1-(4-methoxyphenyl)-2-oxo-4-(2-phenylethenyl)azetidine is dissolved in 65 ml of acetonitrile during a 15 minute period (additional 10 ml of acetonitrile is used for rinse). The mixture is stirred for an additional 15 minutes at 0°C, diluted with 750 ml of ethyl acetate, washed with water (six 600 ml portions), dried over anhydrous sodium sulfate, and stripped of solvent to give an oil. The crude product is chromatographed on 90 g of silica gel, eluting first with 250 ml of 30% ethyl acetate/petroleum ether, then 50% ethyl acetate/petroleum ether. Fractions (50 ml each) 11-16 are combined and evaporated to give 802 mg of the title compound as an oil.

D) (±)-(cis)-3-Azido-2-oxo-4-(2-phenylethenyl)-1-azetidinesulfonic acid, tetra-n-butylammonium salt

(±)-(cis)-3-Azido-2-oxo-4-(2-phenylethenyl)-azetidine (334 mg) is dissolved in 3 ml of dimethylformamide and 868 mg of pyridine-sulfur trioxide is added. The mixture is stirred at room temperature for 40 hours under nitrogen and then poured into 200 ml of 0.5 M monobasic potassium phosphate solution and washed with 30 ml of methylene chloride. tetra-n-Butyl ammonium bisulfate (530 mg) is added to the aqueous solution and the mixture is extracted with methylene chloride (four 50 ml portions). The combined organic layers are back-washed with water (two 100 ml portions), dried over anhydrous magnesium sulfate and stripped of solvent to give 824 mg of the title compound as a gum.

E) (±)-(cis)-3-Azido-2-oxo-4-(2-phenylethenyl)-1-azetidinesulfonic acid

(±)-(cis)-3-Azido-2-oxo-4-(2-phenylethenyl)-1-azetidinesulfonic acid, tetra-n-butylammonium salt (300 mg) is dissolved in 4 ml of tetrahydrofuran and stirred rapidly. To the mixture is added 600 mg of zinc dust followed by 0.8 ml of 1N monobasic potassium phosphate solution. The mixture is heated to 45°C and stirred at this temperature for 3 hours. The mixture is then filtered and the filtrate is taken in 40 ml of methylene chloride and 10 ml of water. The

aqueous layer is further extracted with methylene chloride (three 40 ml portions) and the combined methylene chloride layers are stripped of solvent to give 256 mg of a foam. This crude product is dissolved in a small amount of ca. 30% acetone/water and applied on 7.5 ml of Dowex ($K^+$) resin (0.7 meq./vnl) eluting with 40 ml of water. The eluent is evaporated to give 151 mg of foam, which is dissolved in 2 ml of water and acidified to pH 2 with 1N HCl solution. A small amount of acetonitrile is added to dissolve the precipitate and the resulting solution is applied on 15 ml of HP-20 resin, eluting with 150 ml of water, then 10% acetone/water. Fractions (15 ml each) 2-13 are combined and evaporated to give 101 mg of the title compound as a foam.

## Example 48

(±)-(cis,Z)-3-[[(2-Amino-4-thiazolyl)(methoxyimino)-acetyl]amino]-2-oxo-4-(2-phenylethenyl)-1-azetidine-sulfonic acid, potassium salt

A solution of 68 mg of (Z)-2-amino-α-(methoxyimino)-4-thiazoleacetic acid and 51 mg of N-hydroxybenzotriazole monohydrate in 2 ml of dimethylformamide is treated with 69 mg of dicyclohexylcarbodiimide. The mixture is stirred at room temperature for 30 minutes under nitrogen. (cis)-3-Amino-2-oxo-4-(2-phenylethenyl)-1-azetidinesulfonic acid (90 mg; see example 186) and 34 mg of triethylamine are added and the mixture is stirred for 20 hours under nitrogen.

The slurry is evaporated *in vacuo* and triturated with 10 ml of acetone. The slurry is filtered and the filtrate is treated with 113 mg of potassium perfluorobutanesulfonate. Dilution with 30 ml of ether and filtration gives 169 mg of a solid product, which is dissolved in a small amount of ca. 10% acetonitrile/water and applied on 34 ml of HP-20 resin, eluting with 150 ml of water, then 10% acetone/water. Fractions (15 ml each) 16-19 are combined and stripped of solvent to give 110 mg of the title compound as a solid.

Analysis calc'd for $C_{17}H_{16}O_6N_5S_2K \cdot H_2O$: C, 40.23; H, 3.57; N, 13.80; S, 12.63; K, 7.70
Found: C, 40.03; H, 3.05; N, 13.61; S, 12.31; K, 7.56

Example 49

(cis)-3-Amino-4-(methoxycarbonyl)-2-oxo-1-azetidinesulfonic acid

A) [(4-Methoxyphenyl)imino]acetic acid, methyl ester

A dry 3-necked, 1 liter flask equipped with a nitrogen inlet and stirring bar is charged with 56.88 g of $MgSO_4$ followed by a solution of recrystallized anisidine (19.43 g) in dichloromethane (250 ml). After cooling to $0^{O}C$ a solution of methyl glyoxylate hemiacetal (19.92 g) in dichloromethane (250 ml) is added over 1.5 hours. After stirring an additional 20 minutes at $0^{O}C$,

the reaction mixture is suction filtered, dried over sodium sulfate, filtered and concentrated *in vacuo* to one-quarter volume. Hexane (300 ml) is added, and the solution is concentrated to an oil which semi-solidifies on standing under high vacuum at 5°C.

B) (cis)-3-(1,3-Dihydro-1,3-dioxo-2H-isoindol-2-yl)-4-methoxycarbonyl-2-oxo-1-(4-methoxyphenyl) azetidine

A dry 3-necked 500 ml flask equipped with stirring bar, addition funnel, septum and nitrogen inlet is charged with a solution of [(4-Methoxyphenyl)imino]acetic acid, methyl ester (21.09 g) in dichloromethane (150 ml) and cooled to 0°C. Triethylamine (19.2 ml) 0.14 mole is added dropwise followed by a solution of (N-phthalimido)-acetyl acid chloride (28.4 g) in dichloromethane (150 ml) over 1 hour. The resulting mixture is stirred for 1.5 hours at 0°C, and diluted with 2.5 l of dichloromethane. The organic solution is washed with pH 4.5 monobasic potassium phosphate (two 500 ml portions), 5% sodium bicarbonate (two 500 ml portions), saturated sodium chloride solution (500 ml), and dried over sodium sulfate. Filtration and concentration *in vacuo* yields a solid which is washed with ethyl acetate, cold acetone and hexane to yield 18.65 g of product.

C) (cis)-4-(Methoxycarbonyl)-1-(4-methoxyphenyl)-2-oxo-3-[[(phenylmethoxy)carbonyl]amino]azetidine

A dry 500 ml flask equipped with nitrogen inlet, stirring bar, and septum was charged with 18.65 g of (cis)-3-(1,3-dioxo-2H-isoindol-2-yl)-4-methoxycarbonyl-2-oxo-1-(4-methoxyphenyl)azetidine and 325 ml of dichloromethane. The resulting suspension is cooled to -30°C, and methyl hydrazine (3.52 ml) is added dropwise. The reaction is warmed to 0°C and stirred for 1 hour. An additional 0.4 ml of methyl hydrazine is added and the mixture is stirred for 10 minutes. This sequence is repeated with a total of 2.9 equivalents of methyl hydrazine (7.7 ml) has been added. The solvent was removed *in vacuo*; 200 ml of fresh dichloromethane is added, and the mixture is again concentrated. This sequence was repeated two additional times, the resulting foam was dried under high volume for 20 minutes, redissolved in 225 ml dichloromethane, and allowed to stand at ambient temperature for about 16 hours during which time a considerable amount of solid precipitates. The mixture is filtered under nitrogen, the filtrate cooled to 0°C (nitrogen atmosphere) and treated with diisopropylethyl amine (17 ml) followed by benzyl chloroformate (7 ml) dropwise. The reaction is stirred at 0°C for 30 minutes, then at ambient temperature for 1.5 hours. The mixture is washed with two 300 ml portions of pH 4.5 monobasic potassium phosphate buffer, 5% sodium bicarbonate (two 300 ml portions), saturated sodium chloride (300 ml),

dried (sodium sulfate), and filtered. Concentration in vacuo, yields a foam which on trituration with ether yields 9.9 g of the title compound as a solid.

D) (cis)-4-(Methoxycarbonyl)-2-oxo-3-[[(phenylmethoxy)carbonyl]amino]-1-azetidine

A solution of ceric ammonium nitrate (8.59 g) in 60 ml of 1:1 acetonitrile-water is treated with a slurry of 2 g (cis)-4-(methoxycarbonyl)-1-(4-methoxyphenyl)-2-oxo-3-[[(phenylmethoxy)carbonyl]amino]azetidine in 50 ml acetonitrile over 10 minutes. The reaction mixture is stirred an additional 10 minutes at ambient temperature and diluted with ethyl acetate (100 ml). The separated aqueous layer is washed with ethyl acetate (three 40 ml portions) and the combined organic extracts are washed with 50% sodium bicarbonate (three 70 ml portions). The basic washings are back-washed with ethyl acetate (50 ml) and the combined organic extracts are washed with aqueous sodium sulfite, 5% aqueous sodium carbonate (100 ml), 5% sodium chloride solution (two 100 ml portions), saturated sodium chloride (two 50 ml portions), and stirred over Darco G-60 charcoal for 30 minutes. Sodium sulfate is added, and the mixture is filtered and concentrated in vacuo to yield an oil which on trituration with ether yields 685 mg of the title compound as a solid.

E) (cis)-4-(Methoxycarbonyl)-2-oxo-3-[[(phenylmethoxy)carbonyl]amino]-1-azetidinesulfonic acid, tetrabutylammonium salt

A mixture of (*cis*)-4-(methoxycarbonyl)-2-oxo-3-[[(phenylmethoxy)carbonyl]amino]-1-azetidine (100 mg) and 172 mg of a pyridine-sulfur trioxide complex in 1 ml of pyridine is stirred under argon for 3 hours at 80°C. The reaction mixture is poured into 70 ml of 0.5 M monobasic potassium phosphate (pH 5.5) and extracted with four 30 ml portions of dichloromethane (discard). Tetrabutylammonium hydrogen sulfate (122 mg) is added to the aqueous layer which is then extracted with dichloromethane (four 30 ml portions). The organic extracts are washed with 8% sodium chloride solution, dried over sodium sulfate and filtered. Concentration *in vacuo* yields 186 mg of the title compound as a viscous oil.

F) (cis)-3-Amino-4-(methoxycarbonyl)-2-oxo-1-azetidinesulfonic acid

A solution of 186 mg of (*cis*)-4-(methoxycarbonyl)-2-oxo-3-[[(phenylmethoxy)carbonyl]amino]-1-azetidinesulfonic acid, tetrabutylammonium salt in 2 ml of methanol is hydrogenated over 10% palladium on charcoal (95 mg) for 1.5 hours at 1 atmosphere. The catalyst is filtered off and rinsed with dichloromethane and the filtrate is treated with 97% formic acid and cooled to -50°C (the presence of a seed crystal at this stage is necessary to induce crystallization). After crystallization commences, the mixture is allowed to stand for about 16 hours at 10°C.

The resulting solid is washed with dichloromethane, hexane, and dried in vacuo, yielding 50 mg of the title compound.

Example 50

(cis)-3-[[2-Amino-4-thiazolyl)[[1-(diphenylmethoxycarbonyl)-1-methylethoxy]imino]acetyl]-amino]-4-(methoxycarbonyl)-2-oxo-1-azetidinesulfonic acid, potassium salt

A solution of N-hydroxybenzotriazole hydrate (34 mg) and 101 mg of 2-amino-α-[[1-(diphenylmethoxycarbonyl)-1-methylethoxy]imino]-4-thiazoleacetic acid in 0.5 ml of dimethylformamide is treated with solid dicyclohexylcarbodiimide (45 mg) and the mixture is stirred under argon for 45 minutes (ambient temperature). (cis)-3-Amino-4-(methoxycarbonyl)-2-oxo-1-azetidinesulfonic acid (45 mg; see example 188) is then added as a solid followed by triethylamine (0.03 ml) dropwise. The reaction is stirred at ambient temperature for about 16 hours. The dimethylformamide is removed under high vacuum at 30°C and the residue is triturated with acetone. The supernatant is treated with potassium perfluorobutanesulfonate (67 mg). Dilution with ether produces a solid which is washed with ether and dried in vacuo to yield 93 mg of the title compound.

Example 51

(cis)-3-[[(2-Amino-4-thiazolyl)[(1-carboxy-1-methylethoxy)imino]acetyl]amino]-4-(methoxycarbonyl)-2-oxo-1-azetidinesulfonic acid, dipotassium salt

A slurry of (cis)-3-[[2-Amino-4-thiazolyl)-[[1-(diphenylmethoxycarbonyl)-1-methylethoxy)-imino]acetyl]amino]-4-(methoxycarbonyl)-2-oxo-1-azetidinesulfonic acid, potassium salt in 0.4 ml of anisole is stirred at -12°C under argon, and 0.9 ml of cold (-10°C) trifluoroacetic acid is added. After 1.5 hours, 4 ml of ether and 2 ml of hexane are added and the resulting slurry is stirred for 15 minutes at -10°C, then 15 minutes at ambient temperature. The solid is isolated by centrifugation and washed with ether. The pH of a suspension of this material in 0.5 ml of cold water is adjusted to 6 with 1N potassium hydroxide and then applied to a 30 ml HP-20AG column. Elution with water yields 30 mg of the title compound after evaporation (acetonitrile added and evaporated twice).

Analysis calc'd for $C_{14}H_{15}K_2N_5O_9S_2$: C, 31.15; H, 2.81; N, 12.98

Found: C, 29.08; H, 3.03; N, 12.19

Example 52

[3S]-3-[3,6-dichlorophenylthioacetylamino]-2-oxo-1-azetidinesulfonic acid, potassium salt

Following the procedure of example 4, utilizing 3,6-dichlorophenylthioacetic acid instead of the acid used therein yields the titled compound.

Example 53

(cis)-4-methyl-2-oxo-3-[(3,6-dichlorophenylthioacetyl)-amino]-1-azetidinesulfonic acid, potassium salt

Following the procedure of example 4, utilizing 3,6-dichlorophenylthioacetic acid instead of the acid used therein yields the titled compound.

Example 54

(cis)-4-methyl-2-oxo-3-[(N,N'-diisopropylisothiouronium-acetyl)amino]-1-azetidinesulfonic acid, potassium salt

Following the procedure of example 4, utilizing N,N'-diisopropyl-isothiouronium-acetic acid instead of the acid used therein yields the titled compound.

## Biological Activity

The following methodology is used to determine the minimum inhibitory concentration (hereinafter referred to as MIC) of the β-lactams of this invention.

The test organisms are grown in approximately 15-20 ml of Antibiotic Assay broth (Difco) by inoculating (in tubes) the broth with a loopful of the organism from a BHI (Difco) agar slant. The inoculated tubes are incubated at 37°C for 18 to 20 hours. These cultures are assumed to contain $10^9$ colony forming units (hereinafter CFU) per milliliter. The cultures are diluted 1:100 to give a final inoculum level of $10^4$ CFU; dilutions are made with K-10 broth*.

The compounds are dissolved in the appropriate diluent at a concentration of 1000 μg/ml. Two-fold dilutions are made in K-10 broth resulting in a range from 1000 μg/ml to 0.5 μg/ml. 1.5 ml of each dilution is placed into individual square petri dishes to which 13.5 ml of K-10 agar** is added. The final drug concentration in the agar ranges from 100 μg/ml to 0.05 μg/ml. Organism growth control plates containing agar only are prepared and inoculated before and after the test plates. The organisms are applied to the agar surface of each plate with the Denley Multipoint Inoculator (which delivers approximately 0.001 ml of each organism) resulting in a final inoculum level of $10^4$ CFU on the agar surface.

The plates are incubated at 37°C for 18 hours and the MIC's are determined. The MIC is the lowest concentration of compound inhibiting growth of the organism.

*K-10 broth is a yeast beef broth containing:

| | |
|---|---|
| Beef extract | 1.5 g |
| Yeast extract | 3.0 g |
| Peptone | 6.0 g |
| Dextrose | 1.0 g |
| Distilled water q.s. 1 liter | |

**K-10 agar

| | |
|---|---|
| Beef extract | 1.5 g |
| Yeast extract | 3.0 g |
| Peptone | 6.0 g |
| Dextrose | 1.0 g |
| Agar | 15.0 g |
| Distilled water q.s. 1 liter | |

The tables that follow are tabulated results obtained when the β-lactams of this invention are tested against various organisms. The number following each organism refers to the number of the organism in the collection of E. R. Squibb & Sons, Inc., Princeton, New Jersey. A dash (-) in the tables means that the compound tested did not show activity against the particular organism at 100 μg/ml. The symbol "N.T." means not tested.

M.I.C. (μg/ml)

Product of Example

| Organism | | 1 | 2 | 3 | 4 | 5 | 6 | 7 |
|---|---|---|---|---|---|---|---|---|
| *Staphylococcus aureus,* | 1276 | 100 | 100 | 50 | 25 | 100 | - | 100 |
| *Staphylococcus aureus,* | 2399 | 50 | 100 | 50 | 25 | 100 | - | 100 |
| *Staphylococcus aureus,* | 2400 | 50 | 100 | 50 | 50 | 50 | - | 50 |
| *Staphylococcus aureus,* | 10165 | 100 | - | 100 | 100 | - | - | - |
| *Streptococcus faecalis,* | 9011 | - | - | - | - | 3.1 | 50 | 3.1 |
| *Streptococcus agalactiae,* | 9287 | 100 | 100 | 25 | 12.5 | 3.1 | 12.5 | 6.3 |
| *Micrococcus luteus,* | 2495 | - | 100 | 25 | 50 | 0.1 | 0.4 | 0.2 |
| *Escherichia coli,* | 8294 | - | - | 100 | 100 | <0.05 | <0.05 | <0.05 |
| *Escherichia coli,* | 10857 | - | - | 50 | 50 | 0.1 | 0.2 | 0.2 |
| *Escherichia coli,* | 10896 | - | - | 50 | 100 | <0.05 | <0.05 | 0.1 |
| *Escherichia coli,* | 10909 | - | - | 25 | 50 | 0.2 | 0.4 | 0.4 |
| *Klebsiella aerogenes,* | 10440 | - | - | 100 | - | 0.1 | <0.05 | 0.1 |
| *Klebsiella pneumoniae,* | 9527 | - | - | 100 | 100 | 0.1 | <0.05 | <0.05 |
| *Proteus mirabilis,* | 3855 | - | - | 100 | - | <0.05 | <0.05 | <0.05 |
| *Proteus rettgeri,* | 8479 | - | - | 50 | 100 | <0.05 | <0.05 | <0.05 |
| *Proteus vulgaris,* | 9416 | - | - | 100 | - | <0.05 | <0.05 | <0.05 |
| *Salmonella typhosa,* | 1195 | - | - | 50 | 100 | 0.1 | 0.2 | 0.2 |
| *Shigella sonnei,* | 8449 | - | - | 50 | 100 | 0.2 | 0.4 | 0.2 |
| *Enterobacter cloacae,* | 8236 | - | - | - | - | 0.4 | 0.4 | 0.4 |
| *Enterobacter aerogenes,* | 10078 | - | - | - | - | 0.1 | 0.4 | 0.2 |
| *Citrobacter freundii,* | 9518 | - | - | 100 | - | 0.8 | 0.2 | 0.4 |
| *Serratia marcescens,* | 9783 | - | - | 50 | - | 0.8 | 0.8 | 0.8 |
| *Pseudomonas aeruginosa,* | 9545 | - | - | 50 | - | 100 | 3.1 | - |
| *Pseudomonas aeruginosa,* | 8329 | - | - | - | - | 12.5 | 100 | 50 |
| *Acinetobacter calcoaceticus,* | 8333 | - | - | - | - | | | |

M.I.C. (μg/ml)

| Organism | | Product of Example 9 | 11 | 12 | 13 | 14 | 15 | 16 |
|---|---|---|---|---|---|---|---|---|
| *Staphylococcus aureus,* | 1276 | 100 | 25 | 25 | 25 | 50 | 6.3 | - |
| *Staphylococcus aureus,* | 2399 | 50 | 25 | 50 | N.T. | 50 | 6.3 | - |
| *Staphylococcus aureus,* | 2400 | 50 | 25 | 50 | 12.5 | 100 | 12.5 | - |
| *Staphylococcus aureus,* | 10165 | - | - | - | 25 | - | 50 | - |
| *Streptococcus faecalis,* | 9011 | - | - | - | - | - | - | - |
| *Streptococcus agalactiae,* | 9287 | 1.6 | 0.4 | 6.3 | 25 | 3.1 | 0.8 | 50 |
| *Micrococcus luteus,* | 2495 | 6.3 | 6.3 | 6.3 | 3.1 | 12.5 | 1.6 | 25 |
| *Escherichia coli,* | 8294 | 0.1 | 1.6 | 1.6 | 1.6 | 1.6 | 50 | 0.4 |
| *Escherichia coli,* | 10857 | <0.05 | <0.05 | <0.05 | 1.6 | 0.4 | 0.4 | 0.2 |
| *Escherichia coli,* | 10896 | 0.1 | 0.4 | 1.6 | 1.6 | 1.6 | 6.3 | 0.2 |
| *Escherichia coli,* | 10909 | <0.05 | 0.1 | 0.1 | 0.8 | 0.8 | 6.3 | <0.05 |
| *Klebsiella aerogenes,* | 10440 | 0.2 | 0.8 | 1.6 | 1.6 | 3.1 | - | 0.8 |
| *Klebsiella pneumoniae,* | 9527 | <0.05 | 0.1 | 0.1 | 1.6 | 1.6 | 12.5 | 0.1 |
| *Proteus mirabilis,* | 3855 | <0.05 | 0.4 | 0.4 | 1.6 | 0.8 | 6.3 | <0.05 |
| *Proteus rettgeri,* | 8479 | <0.05 | 0.4 | 0.4 | 0.1 | 0.8 | 1.6 | <0.05 |
| *Proteus vulgaris,* | 9416 | <0.05 | <0.05 | <0.05 | 0.8 | 0.8 | 1.6 | <0.05 |
| *Salmonella typhosa,* | 1195 | <0.05 | 0.2 | 0.2 | 0.8 | 0.8 | 6.3 | <0.05 |
| *Shigella sonnei,* | 8449 | 0.1 | 0.4 | 0.8 | 1.6 | 0.8 | 25 | 0.4 |
| *Enterobacter cloacae,* | 8236 | 0.1 | 0.8 | 1.6 | 1.6 | 3.1 | 25 | 0.8 |
| *Enterobacter aerogenes,* | 10078 | 0.2 | 1.6 | 3.1 | 3.1 | 3.1 | 100 | 0.8 |
| *Citrobacter freundii,* | 9518 | 0.1 | 0.8 | 1.6 | 0.8 | 1.6 | 25 | 0.8 |
| *Serratia marcescens,* | 9783 | 0.2 | 0.8 | 1.6 | 0.8 | 3.1 | 50 | 0.4 |
| *Pseudomonas aeruginosa,* | 9545 | 0.4 | 1.6 | 0.8 | 6.3 | 3.1 | 12.5 | 0.8 |
| *Pseudomonas aeruginosa,* | 8329 | 100 | 12.5 | 12.5 | - | 100 | - | 3.1 |
| *Acinetobacter calcoaceticus,* | 8333 | 25 | 12.5 | 12.5 | 12.5 | 100 | - | 50 |

M.I.C. (μg/ml)

Product of Example

| Organism | | 20 | 21 | 22 | 23 | 25 | 26 | 27 |
|---|---|---|---|---|---|---|---|---|
| *Staphylococcus aureus*, | 1276 | 6.3 | 3.1 | 6.3 | - | - | - | - |
| *Staphylococcus aureus*, | 2399 | 6.3 | 3.1 | 6.3 | - | - | - | - |
| *Staphylococcus aureus*, | 2400 | 25 | 6.3 | 25 | - | - | - | - |
| *Staphylococcus aureus*, | 10165 | - | 100 | 100 | - | - | - | - |
| *Streptococcus faecalis*, | 9011 | - | - | - | - | - | - | - |
| *Streptococcus agalactiae*, | 9287 | 3.1 | 0.4 | 6.3 | 12.5 | - | - | 12.5 |
| *Micrococcus luteus*, | 2495 | 3.1 | 3.1 | 6.3 | 12.5 | - | - | 100 |
| *Escherichia coli*, | 8294 | 3.1 | 1.6 | 3.1 | 0.2 | 50 | - | 6.3 |
| *Escherichia coli*, | 10857 | <0.05 | <0.05 | 0.1 | 0.1 | 6.3 | 50 | 3.1 |
| *Escherichia coli*, | 10896 | 0.8 | 0.4 | 1.6 | 0.2 | 25 | - | 3.1 |
| *Escherichia coli*, | 10909 | 0.4 | 0.2 | 0.4 | N.T. | 25 | - | 1.6 |
| *Klebsiella aerogenes*, | 10440 | 12.5 | 1.6 | 12.5 | 0.4 | 100 | - | 6.3 |
| *Klebsiella pneumoniae*, | 9527 | 0.4 | 0.2 | 0.1 | 0.05 | 6.3 | - | 6.3 |
| *Proteus mirabilis*, | 3855 | 1.6 | 0.8 | 0.4 | 0.05 | 12.5 | - | 3.1 |
| *Proteus rettgeri*, | 8479 | 1.6 | 1.6 | 0.2 | 0.05 | 0.8 | 100 | 0.4 |
| *Proteus vulgaris*, | 9416 | <0.05 | <0.05 | <0.05 | 0.05 | 12.5 | 100 | 6.3 |
| *Salmonella typhosa*, | 1195 | 1.6 | 0.4 | 0.4 | 0.05 | 12.5 | - | 3.1 |
| *Shigella sonnei*, | 8449 | 1.6 | 0.8 | 1.6 | 0.1 | 25 | - | 3.1 |
| *Enterobacter cloacae*, | 8236 | 1.6 | 1.6 | 0.8 | 0.1 | 100 | - | 3.1 |
| *Enterobacter aerogenes*, | 10078 | 6.3 | 3.1 | 6.3 | 0.8 | 100 | - | 6.3 |
| *Citrobacter freundii*, | 9518 | 3.1 | 1.6 | 3.1 | 0.4 | 25 | - | 3.1 |
| *Serratia marcescens*, | 9783 | 3.1 | 3.1 | 6.3 | 0.2 | 100 | - | 3.1 |
| *Pseudomonas aeruginosa*, | 9545 | 3.1 | 12.5 | 6.3 | 0.4 | - | - | 50 |
| *Pseudomonas aeruginosa*, | 8329 | 100 | 12.5 | 12.5 | 1.6 | - | - | - |
| *Acinetobacter calcoaceticus*, | 8333 | 50 | 50 | 12.5 | 25 | - | - | - |

| Organism | | M.I.C. (μg/ml) Product of Example 29 | 31 | 32 |
|---|---|---|---|---|
| *Staphylococcus aureus*, | 1276 | - | - | 25 |
| *Staphylococcus aureus*, | 2399 | - | - | 12.5 |
| *Staphylococcus aureus*, | 2400 | - | - | 25 |
| *Staphylococcus aureus*, | 10165 | - | - | - |
| *Streptococcus faecalis*, | 9011 | - | - | - |
| *Streptococcus agalactiae*, | 9287 | - | 100 | 6.3 |
| *Micrococcus luteus*, | 2495 | - | - | 6.3 |
| *Escherichia coli*, | 8294 | - | 0.4 | 3.1 |
| *Escherichia coli*, | 10857 | - | 0.2 | 0.4 |
| *Escherichia coli*, | 10896 | 100 | 0.1 | 12.5 |
| *Escherichia coli*, | 10909 | 100 | 0.1 | 0.8 |
| *Klebsiella aerogenes*, | 10440 | - | 0.8 | 1.6 |
| *Klebsiella pneumoniae*, | 9527 | 100 | 0.2 | 0.2 |
| *Proteus mirabilis*, | 3855 | - | 0.2 | 0.4 |
| *Proteus rettgeri*, | 8479 | - | 0.05 | 0.4 |
| *Proteus vulgaris*, | 9416 | - | 0.2 | 0.2 |
| *Salmonella typhosa*, | 1195 | - | 0.2 | 0.2 |
| *Shigella sonnei*, | 8449 | - | 0.4 | 6.3 |
| *Enterobacter cloacae*, | 8236 | - | 0.2 | 6.3 |
| *Enterobacter aerogenes*, | 10078 | - | 0.4 | 6.3 |
| *Citrobacter freundii*, | 9518 | - | 0.2 | 3.1 |
| *Serratia marcescens*, | 9783 | - | 0.4 | 3.1 |
| *Pseudomonas aeruginosa*, | 9545 | - | 0.8 | 6.3 |
| *Pseudomonas aeruginosa*, | 8329 | - | 12.5 | 50 |
| *Acinetobacter calcoaceticus*, | 8333 | - | 100 | 50 |

CLAIMS

1. A process for preparing a β-lactam having a sulfonic acid salt substituent $-SO_3^-M^+$ wherein $M^+$ is hydrogen or a cation, in the 1-position and an amino substituent $-NH_2$ or a protected form thereof in the 3-position and having one or two alkyl, cycloalkyl, phenyl or substituted phenyl substituents in the 4-position or a single alkoxycarbonyl, alken-1-yl, alkyn-1-yl, 2-phenylethenyl or 2-phenylethynyl substituent in the 4-position by sulfonating a corresponding β-lactam having a hydrogen substituent in the 1-position and a protected amino substituent in the 3-position and removing said amino protecting group.

2. A process according to claim 1 wherein the amino substituent in the 3-position is acylated, said acyl group containing a basic function when $M^+$ is hydrogen and said acylation is effected either before or after the sulfonation reaction.

3 . A process according to claims 1 and 2 wherein the product contains an alkyl substituent in the 4-position.

4. A process according to claims 1 through 3 wherein the product is of the formula

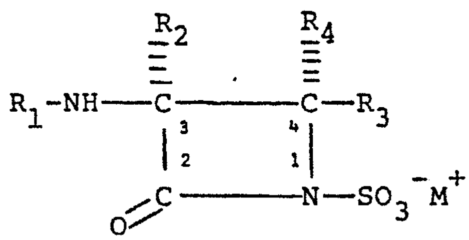

wherein $R_1$ is hydrogen or acyl or the group $R_1$-NH- is protected amino; $R_2$ is hydrogen or $C_1$-$C_4$ alkoxy; $R_3$ and $R_4$ are the same or different and each is alkyl, cycloalkyl, phenyl or substituted phenyl, or one or $R_3$ and $R_4$ is hydrogen and the other is alkyl, cycloalkyl, phenyl, substituted phenyl, alkoxycarbonyl, alken-1-yl, alkyn-1-yl, 2-phenylethenyl or 2-phenylethynyl; and $M^+$ is hydrogen or a cation and said sulfonation is effected on a compound of the formula

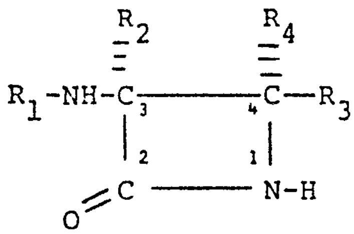

or a precursor compound of the formula

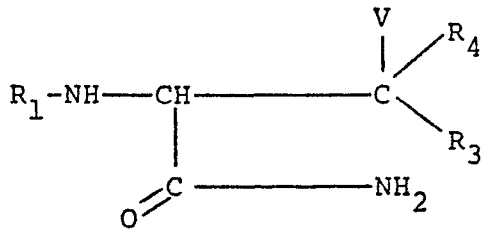

which is cyclized, said sulfonation reaction being effected either before or after said cyclization, $R_1$-NH- in the reactants being a protected amino group or $R_1$ being acyl; V being a leaving group such as methanesulfonyl, benzenesulfonyl, toluenesulfonyl, chloro, bromo or iodo; and $R_2$, $R_3$ and $R_4$ being as previously defined.

5. A process according to claim 4 wherein $R_1$ is hydrogen or the group $R_1$-NH- is a protected amino group.

6. A process according to claim 4 wherein $R_1$ is acyl.

7. A process according to claim 6 wherein the acyl group is selected from the group consisting of (a) an aliphatic group having the formula

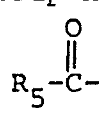

wherein $R_5$ is alkyl; cycloalkyl; alkoxy, alkenyl; cycloalkenyl, cyclohexadienyl; or alkyl or alkenyl substituted with one or more halogen, cyano, nitro, amino, mercapto, alkylthio, or cyanomethylthio groups; (b) a carbocyclic aromatic group having the formula

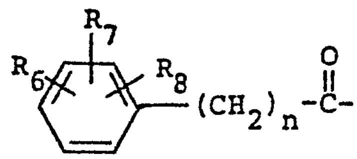
,

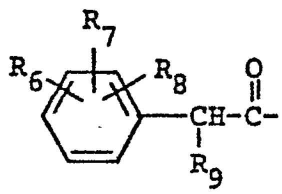

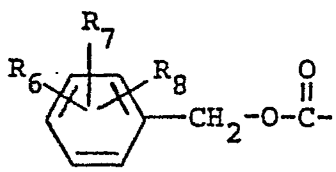

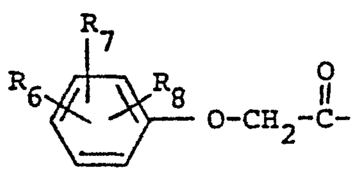

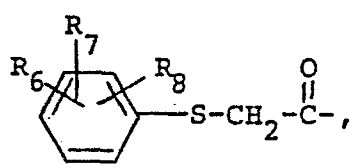
, or

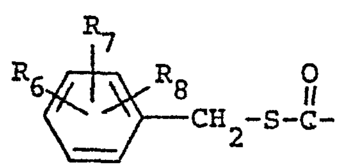

wherein n is 0, 1, 2 or 3; $R_6$, $R_7$ and $R_8$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_9$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio;

(c) a heteroaromatic group having the formula

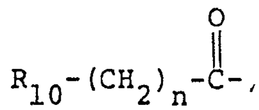

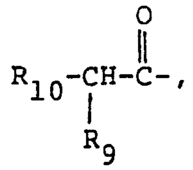

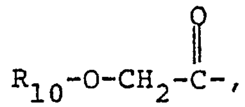

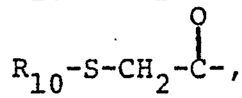 or

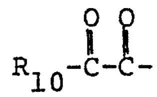

wherein n is 0, 1 2 or 3; $R_9$ is amino, hydroxyl, a carboxyl salt, protected carboxyl, formyloxy, a sulfo salt, a sulfoamino salt, azido, halogen, hydrazino, alkylhydrazino, phenylhydrazino or [(alkylthio)thioxomethyl]thio; and $R_{10}$ is a substituted or unsubstituted 5-, 6- or 7-membered heterocyclic aromatic ring containing 1, 2, 3 or 4 nitrogen, oxygen and sulfur atoms;

(d) a group having the formula

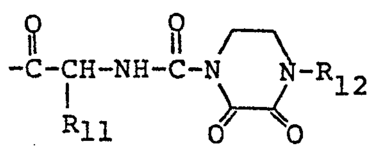

wherein $R_{11}$ is (i) a group having the formula

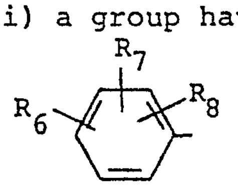

wherein $R_6$, $R_7$ and $R_8$ each is independently hydrogen, halogen, hydroxy, nitro, amino, cyano, trifluoromethyl, alkyl or 1 to 4 carbon atoms, alkoxy of 1 to 4 carbon atoms, or aminomethyl, or (ii) a substituted or unsubstituted 5-, 6- or 7-membered heterocyclic ring containing 1, 2, 3 or 4 nitrogen, oxygen and sulfur atoms; and $R_{12}$ is $-N{=}CH-R_{11}$, $-NH-\overset{O}{\overset{\|}{C}}-R_{11}$, alkylcarbonylamino, alkyl or alkyl substituted with one or more halogen, cyano, nitro, amino or mercapto groups;

(e) a group having the formula

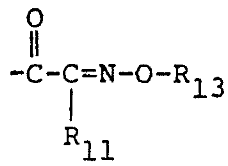

wherein $R_{11}$ is (i) a group having the formula

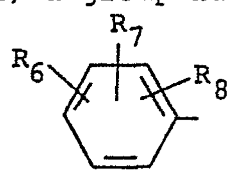

wherein $R_6$, $R_7$ and $R_8$ each is independently hydrogen, halogen, hydroxyl, nitro, amino, cyano, trifluoromethyl, alkyl of 1 to 4 carbon atoms, alkoxy of 1 to 4 carbon atoms or aminomethyl, or (ii) a substituted or unsubstituted 5-, 6- or 7-membered heterocyclic ring containing 1, 2, 3 or 4 nitrogen, oxygen and sulfur atoms; and $R_{13}$ is hydrogen, alkyl, cycloalkyl, alkylaminocarbonyl, $-\overset{O}{C}-NH-R_{11}$, or alkyl substituted with one or more halogen, cyano, nitro, amino, mercapto, alkylthio, $R_{11}$, carboxyl, carboxyl salt, amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl or dialkoxyphosphinyl substituents;

(f) a group having the formula

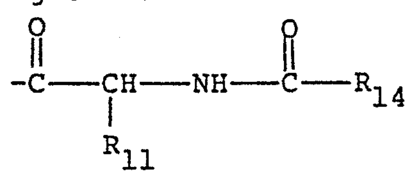

wherein $R_{11}$ is (i) a group having the formula

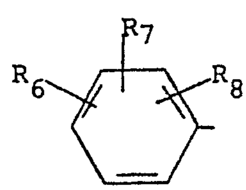

0187355

wherein $R_6$, $R_7$ and $R_8$ each is independently hydrogen, halogen, hydroxyl, nitro, amino, cyano, trifluoromethyl, alkyl of 1 to 4 carbon atoms, alkoxy of 1 to 4 carbon atoms, or aminomethyl, or (ii) a substituted or unsubstituted 5-, 6- or 7-membered heterocyclic ring containing 1, 2, 3 or 4 nitrogen, oxygen and sulfur atoms; and $R_{14}$ is

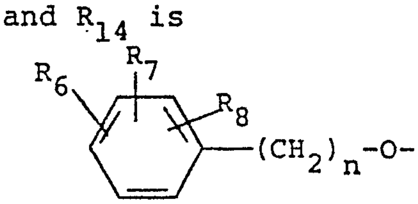

wherein n is 0, 1, 2 or 3, amino, alkylamino, (cyanoalkyl)amino, amido, alkylamido, (cyanoalkyl)amido,

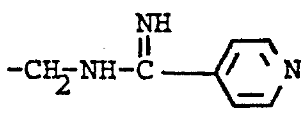 , 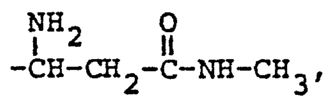,

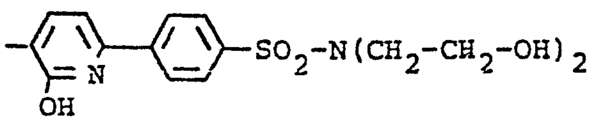 , 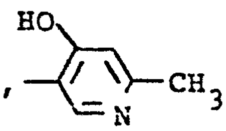

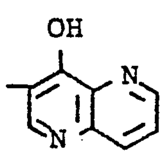 , or 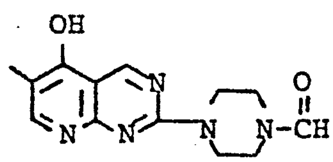 .

(g) a group having the formula

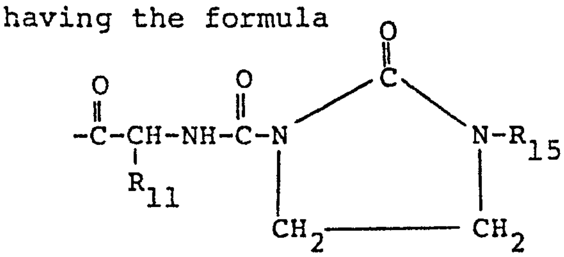

wherein the $R_{11}$ is (i) a group having the formula

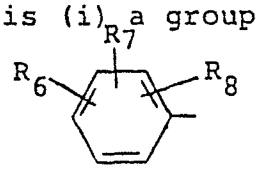

wherein $R_6$, $R_7$ and $R_8$ each is independently hydrogen, halogen, hydroxyl, nitro, amino, cyano, trifluoromethyl, alkyl of 1 to 4 carbon atoms, alkoxy of 1 to 4 carbon atoms, or aminomethyl, or (ii) a substituted or unsubstituted 5-, 6- or 7-membered heterocyclic ring containing 1, 2, 3 or 4 nitrogen, oxygen and sulfur atoms; and $R_{15}$ is hydrogen, alkylsulfonyl, $-N{=}CH-R_{11}$, $-\overset{O}{\overset{\|}{C}}-R_{16}$ wherein $R_{16}$ is hydrogen, alkyl, or halogen substituted alkyl, $R_{11}$, alkyl, or alkyl substituted with one or more halogen, cyano, nitro, amino or mercapto groups.

8. A process in accordance with claim 7 wherein $R_{11}$ is 2-amino-4-thiazolyl.

9. A process in accordance with claim 8 wherein the product is a salt of [3S-[3α(Z),4β]]-3-[[(2-amino-4-thiazolyl)-methoxyimino)acetyl]amino]-4-methyl-2-oxo-1-azetidinesulfonic acid.

10. A process in accordance with claim 8 wherein the product is a salt of [3S-[ 3α(Z), 4β[[-3-[[[carboxy-methoxy)-imino](2-amino-4-thiazolyl)acetyl]amino]-4-methyl-2-oxo-1-azetidinesulfonic acid.

11. A process in accordance with claim 8 wherein the product is a salt of [3S-[3α(Z), 4β]]-3-[[(2-amino-4-thiazolyl)-[(1-carboxy-1-methylethoxy)imino]acetyl]-amino]-4-methyl-2-oxo-1-azetidinesulfonic acid.

12. A process in accordance with claim 11 wherein the product is [3S-[3α(Z),4β]]-3-[[(2-amino-4-thiazolyl)-[(1-carboxy-1-methylethoxy)imino]acetyl]amino]-4-methyl-2-oxo-1-azetidinesulfonic acid, dipotassium salt.

13. A process in accordance with claim 8 wherein the product is a salt of [3S- [3α(Z),4α]]-3-[[2-amino-4-thiazolyl)-methoxyimino)acetyl]amino]-4-methyl-2-oxo-1-azetidinesulfonic acid.

14. A process in accordance with claim 8 wherein the product is a salt of [3S-[3α(Z),4α]]-3-[[(2-amino-4-thiazolyl)-[(1-carboxy-1-methylethoxy)imino]acetyl]-amino]-4-methyl-2-oxo-1-azetidinesulfonic acid.

15. A process in accordance with claim 7 wherein the product is a salt of [3S-[3α(R*)4β]]-3-[[[[[3-[(2-furanylmethylene)amino]-2-oxo-1-imidazolidinyl]carbonyl]-amino]phenylacetyl]amino]-4-methyl-2-oxo-1-azetidine-sulfonic acid.

16. A process in accordance with claim 7 wherein the product is a salt of [3S-[3α(R*),4α]]-3-[[[[[3-[(2-furanylmethylene)amino]-2-oxo-1-imidazolidinyl]carbonyl]-amino]-henylacetyl]amino]-4-methyl-2-oxo-1-azetidine-sulfonic acid.

17. A process in accordance with claim 7 wherein the product is a salt of [3S-[3α(R*),4β]]-3-[[[[(4-ethyl-2,3-dioxo-1-piperazinyl)carbonyl]amino]phenylacetyl]amino]-4-methyl-2-oxo-1-azetidinesulfonic acid.

18. A process in accordance with claim 7 wherein the product is a salt of [3S-[3α(R*),4α]]-3-[[[[(4-ethyl-2,3-dioxo-1-piperazinyl)carbonyl]amino]phenylacetyl]amino]-4-methyl-2-oxo-1-azetidinesulfonic acid.

19. A process according to the preceding claims wherein the sulfonation is effected by treatment with a sulfur trioxide complex or an equivalent sulfonating reagent.

20. A process according to claim 19 wherein the sulfonating complex is pyridine sulfur trioxide complex.

21. A process according to claim 19 wherein the sulfonating complex is dimethylformamide-sulfur trioxide complex.

22. A process according to claim 1 wherein the amino substituent in the 3-position is in the protected form of an azide.

23. A process according to claim 4 wherein the group $R_1$-NH is an amino group in the protected form of an azide.

24. A process according to claims 22 and 23 wherein the azide protecting group is reduced to form the amino substituent ($-NH_2$).

25. A process according to claims 1 and 4 wherein the amino protecting group is benzyloxycarbonyl or butyloxycarbonyl.

26. A process according to claims 1, 3 and 4 wherein the product is of the formula

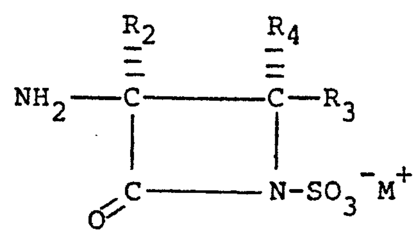

wherein the various groups are as defined in claim 4 and the amino group can be in a protected form.

27. A process according to claim 4 wherein $R_2$ is hydrogen and one of $R_3$ and $R_4$ is hydrogen and the other is alkyl.

28. A process according to claim 27 wherein the alkyl group is methyl.

29. A process according to the preceding claims wherein the cation $M^+$ is a pyridinium ion or a tetrabutylammonium ion or a potassium ion.

30. A compound of the formula

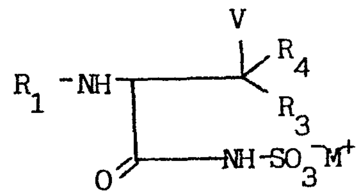

wherein $R_1$, $R_3$. $R_4$, and M are as defined in claim 4, and V is a leaving group such as methanesulfonyl, benzenesulfonyl, toluenesulfonyl, chloro, bromo or iodo.

31. A process for preparing a compound of the formula

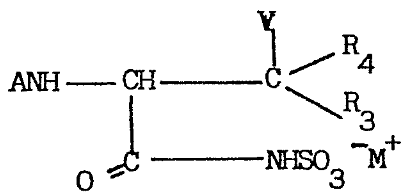

wherein A is a protecting group and V, $R_3$ and $R_4$ and M are as defined in claim 30 which comprises sulfonating a compound of the formula

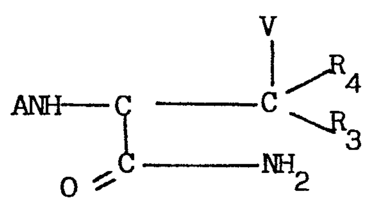

wherein the symbols are as defined above.

32. A compound of the formula

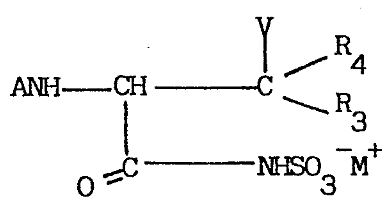

wherein the symbols are as defined in claim 31.

European Patent Office

# EUROPEAN SEARCH REPORT

0187355 Application number

EP 85 11 6364

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 021 678 (TAKEDA)<br>* Claims *<br>--- | 1-8 | C 07 D 205/08<br>C 07 D 417/12<br>C 07 C 143/86<br>C 07 C 125/065<br>C 07 D 405/14<br>C 07 D 403/12<br>C 07 D 409/12 |
| Y | JOURNAL OF THE AMERICAN CHEMICAL SOCIETY, vol. 102, no. 23, 5th November 1980, pages 7026-7032, American Chemical Society, US; M.J. MILLER et al.: "Synthesis of beta-lactams from substituted hydroxamic acids"<br>* Whole article *<br>--- | 1-8,30-32 | |
| E | EP-A-0 053 816 (TAKEDA)<br>* Claims *<br>----- | 1-29 | |
| | | | **TECHNICAL FIELDS SEARCHED (Int. Cl.4)** |
| | | | C 07 D 205/00<br>C 07 D 417/00<br>C 07 D 403/00<br>C 07 D 405/00<br>C 07 D 409/00<br>C 07 C 143/00<br>C 07 C 125/00 |

The present search report has been drawn up for all claims

| Place of search | Date of completion of the search | Examiner |
|---|---|---|
| THE HAGUE | 24-03-1986 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82